(19) Europäisches Patentamt
European Patent Office
Office européen des brevets

(11) EP 3 466 405 B1

(12) # EUROPEAN PATENT SPECIFICATION

(45) Date of publication and mention of the grant of the patent:
**28.06.2023 Bulletin 2023/26**

(21) Application number: **17806385.5**

(22) Date of filing: **17.05.2017**

(51) International Patent Classification (IPC):
***A61K 8/898*** *(2006.01)* ***A61K 8/11*** *(2006.01)*
***A61Q 17/04*** *(2006.01)* ***A61Q 19/00*** *(2006.01)*
***A61Q 1/06*** *(2006.01)*

(52) Cooperative Patent Classification (CPC):
**A61K 8/11; A61K 8/898; A61Q 19/00;**
A61K 2800/10; A61Q 1/06; A61Q 17/04

(86) International application number:
**PCT/JP2017/018585**

(87) International publication number:
**WO 2017/208827 (07.12.2017 Gazette 2017/49)**

(54) **MICROCAPSULES AND COSMETIC COMPRISING THEM**

MIKROKAPSELN UND DIESE ENTHALTENDES KOSMETIKUM

MICROCAPSULES ET COSMÉTIQUE LES COMPRENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.06.2016 JP 2016111895**

(43) Date of publication of application:
**10.04.2019 Bulletin 2019/15**

(73) Proprietor: **Seiwa Kasei Company, Limited**
**Higashiosaka-shi, Osaka 579-8004 (JP)**

(72) Inventors:
- **YOSHIOKA Masato**
**Higashiosaka-shi**
**Osaka 579-8004 (JP)**
- **TOMIHISA Shota**
**Higashiosaka-shi**
**Osaka 579-8004 (JP)**
- **HOMMA Yuta**
**Higashiosaka-shi**
**Osaka 579-8004 (JP)**

(74) Representative: **Vossius & Partner**
**Patentanwälte Rechtsanwälte mbB**
**Siebertstrasse 3**
**81675 München (DE)**

(56) References cited:
**JP-A- H0 867 608** **JP-A- 2001 049 233**
**JP-A- 2001 334 143** **JP-A- 2012 505 742**
**JP-A- 2013 538 683** **JP-B1- 6 023 947**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 466 405 B1

## Description

### TECHNICAL FIELD

The present invention relates to a microcapsule containing core material and to a cosmetic containing the microcapsules containing core material for use in cosmetics, quasi-drugs, drugs, etc. More particularly, the present invention relates to a microcapsule containing core material which is easy to manufacture, causing almost no leaching of the core material, exhibiting extremely high stability where almost no precipitation occurs by aggregation of the capsules, and generating almost no odor from the capsule wall; and to a cosmetic containing the same.

### BACKGROUND ART

Conventionally, microcapsules in which a drug and the like are contained are widely used in cosmetics and in pharmaceuticals. In the cosmetics field, for example, they have been used for the purposes to prevent direct contact between skin and an active ingredient which may cause inflammation in skin, by encapsulating the ingredient into the microcapsules (Patent Document 1), and to exert long-term effects of the component such as flavor to be released gradually from the capsules (Patent Document 2) .

As the material constituting the wall membrane of the capsule (wall material), proteins, natural substances such as polysaccharides, and acrylic polymers, as well as biostable silicone materials have been used. The present inventors have developed a microcapsule containing an oily substance such as an ultraviolet absorber in which a co-polycondensate of silylated peptide and silane compound was used as wall material (Patent Documents 3 and 4). Since the microcapsule using the co-polycondensate of silylated peptide and silane compound as wall material has dense capsule wall, causes less leaching of core material and is excellent in stability over time, it has been widely used in the cosmetics field.

In the cosmetics field, in the case of encapsulation for the purpose to prevent direct contact between skin and active ingredient, it is necessary to construct capsule wall enough to prevent leaching of the contained ingredient from the capsule. On the other hand, when containing the substance such as ultraviolet absorber, constructing excessively thick wall will deteriorate ultraviolet absorbing ability or the like, and the absorbent will not exert its capability sufficiently. In skin cosmetics, when the capsule particle diameter is large, or the distribution of the particle size of the capsule is wide, problems such as giving foreign body feeling when applied to skin, or makeup float (white float) may occur.

Furthermore, the use of natural polymers such as polysaccharides, proteins and their hydrolysates as the wall material of the capsule may cause problems such as giving sticky feeling (stickiness) to hair and skin, depending on external humidity, or generating odor from the wall material. Furthermore, the microcapsules described in Patent Documents 3 and 4, which utilize co-polycondensates of silylated peptide and silane compound as wall material, have following problems, since the peptide portion of the silylated peptide was derived from natural protein.
Patent Document 5 relates to an ultraviolet absorbing composition comprising microcapsules containing an ultraviolet absorbent and a substance having a property of scattering ultraviolet rays.

For example, natural proteins often have an isoelectric point at acidic pH and peptides produced by hydrolyzation of the natural proteins are prone to aggregation and precipitation at the pH, or aggregation by associating with cationic substances combined in cosmetics. Remaining odor in final formulation which comes from the raw materials or generated upon hydrolysis of proteins is also a problem. The extent of hydrolysis of proteins must be controlled depending on the proteins in the preparation of peptides, and the molar ratio of silylated peptide and silane compound in the co-polycondensation reaction must be carefully determined in advance, making the manufacture of the microcapsules complicated. Therefore, development of a method which solves the above problems and makes the manufacture of microcapsules easier has been desired.

### PRIOR ART REFERENCES

### PATENT DOCUMENTS

Patent Document 1: JP-A-2002-037713
Patent Document 2: JP-A-10-277512
Patent Document 3: JP-A-11-221459
Patent Document 4: JP-A-2000-225332
Patent Document 5: JP-A-2001-049233

## SUMMARY OF THE INVENTION

## SUBJECTS TO BE SOLVED BY THE INVENTION

An object of the present invention is to provide a microcapsule that contains core material causing less leaching of the core material, generating almost no odor from wall material, having high stability in wide pH range, and not causing aggregation by associating with substance combined with in cosmetic formulations, yet core materials can exhibit their activity sufficiently.

Another object of the present invention is to provide a cosmetic comprising the microcapsules containing core material as a cosmetic substrate.

## MEANS FOR SOLVING THE SUBJECTS

The present inventors have conducted extensive studies to solve the above subjects, and, as a result, have found that;

a microcapsule containing core material such as a drug or the like, having a wall material made of a copolymer of silylated amino acid and silane compound which is easy to manufacture by co-polycondensation of a silylated amino acid and a silane compound, and the microcapsule thus manufactured has good stability, causes almost no leaching of core material, has good stability over time without causing aggregation and precipitation even at low pH or in the presence of cationic substances, and generates almost no odor from the wall material; and have completed the present invention.

The present invention provides, as a first embodiment thereof,

a microcapsule containing core material in which
the capsule wall is made of a silylated amino acid/silane compound copolymer which has a structural unit U represented by the following general formula (Ia), (Ib) or (Ic):

[Formula 1]

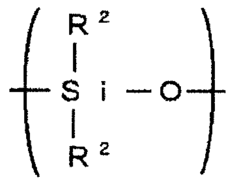

(Ia)

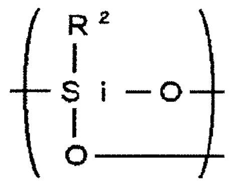

(Ib)

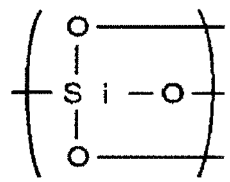

(Ic)

, wherein, $R^2$ represents an alkyl group having 1 to 20 carbon atoms, each $R^2$ may be the same or different, and a structural unit W represented by the following general formula (Id) or (Ie):

[Formula 2]

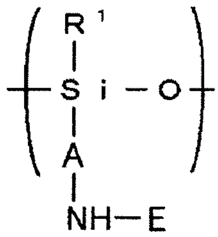

(Id)

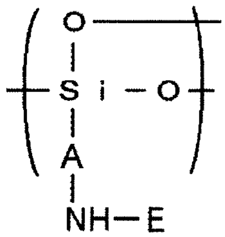

(Ie)

, wherein, $R^1$ represents an alkyl group having 1 to 3 carbon atoms, each $R^1$ may be the same or different, A is a divalent group connecting Si and N and is at least one group selected from the group consisting of $-CH_2-$, $-CH_2CH_2-$, $-CH_2CH_2CH_2-$, $*-(CH_2)_3OCH_2CH(OH)CH_2-$ and $*-(CH_2)_3OCOCH_2CH_2-$(* indicates the side that bonds to Si), and E is a residue obtained by removing one primary amino group from an $\alpha$ amino acid (claim 1).

Note that when E represents a residue obtained by removing one primary amino group from an $\alpha$ amino acid which has even other amino group other than the $\alpha$-amino group (a basic amino acid such as lysine), the primary amino group to be removed may be either the $\alpha$-amino group or the other amino group. Further, in this case, N of the amino group remaining in E may be bonded to A in the other structural units W.

The present invention also provides, as preferred embodiments of the first embodiment;

a microcapsule containing core material of the first embodiment, in which the structural unit U is represented by formula (Ia) or (Ib) and the structural unit W is represented by formula (Ie) (claim 2);
a microcapsule containing core material of the first embodiment, in which the $\alpha$ amino acid is a hydrophilic amino acid (claim 3); and
a microcapsule containing core material of the first embodiment, in which a group represented by the following general formula (II) is bonded to the end of the silylated amino/silane compound copolymer (claim 4).

[Formula 3]

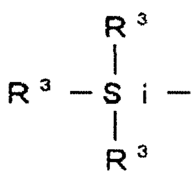

(II)

, wherein, $R^3$ represents an alkyl group having 1 to 4 carbon atoms or a phenyl group, each $R^3$ may be the same or different.

The microcapsule containing core material in which a group represented by the general formula (II) is bonded to the end of the silylated amino/silane compound copolymer can be produced by performing a surface treatment of the capsule for preventing aggregation which will be described below.

The present invention provides, as a second embodiment thereof,

a microcapsule containing core material

which has capsule wall made of a silylated amino acid/silane compound copolymer obtained by co-polycondensation of

a silylated amino acid in which a silyl group represented by the following general formula (III):

[Formula 4]

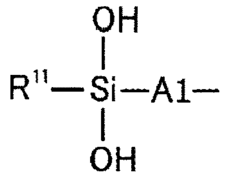 (III)

wherein, $R^{11}$ represents a hydroxyl group or an alkyl group having 1 to 3 carbon atoms, and A1 represents a connecting group selected from a group consisting of $-CH_2-$, $-CH_2CH_2-$, $-CH_2CH_2CH_2-$, $*-(CH_2)_3OCH_2CH(OH)CH_2-$ and $*-(CH_2)_3OCOCH_2CH_2-$ (* indicates a side that bonds to Si), is bonded to an amino group of an $\alpha$-amino acid and

a silane compound represented by the following general formula (IV):

$$R^{21}{}_mSi(OH)_nY_{(4-n-m)} \qquad (IV)$$

, wherein, $R^{21}$ represents an alkyl group having 1 to 20 carbon atoms or a phenyl group, m is an integer from 0 to 3, all the m of $R^{21}$ may be the same or different, n is an integer from 0 to 4, $m+n \leqq 4$, (4-n-m) of Y represent a hydrogen or an alkoxy group having 1 to 6 carbon atoms,

in a dispersion in which a phase of an oily substance to become core material is dispersing in a continuous phase constituted of an aqueous substance, or

in a dispersion in which a phase of an aqueous substance to become core material is dispersing in a continuous phase constituted of an oily substance; and

which contains the above-core material (claim 6).

In the general formula (IV), n is preferably an integer from 2 to 4.

The microcapsule containing core material of the first embodiment can be prepared by the same procedure as the microcapsule containing core material of the second embodiment. That is, the microcapsule containing core material of the second embodiment is a microcapsule containing core material of the first embodiment defined by its manufacturing process.

In the co-polycondensation, the silylated amino acid in which a silyl group represented by the general formula (III) is bonded is used in an amount of 0.1 to 40 times molar amounts preferably, more preferably 1 to 20 times molar amounts, of the silane compound represented by formula (IV).

In the co-polycondensation of the silylated amino acid and the silane compound represented by general formula (IV) for manufacturing microcapsules containing core material of the second embodiment, the capsule walls are formed at the interface between the continuous phase and the dispersed phase in the dispersion and microcapsules containing the dispersed phase as the core material are generated. Therefore, when the raw material for capsule wall is a compound having a hydrophilic portion and a hydrophobic portion, the co-polycondensation at the interface tends to occur easily, and microcapsules having higher stability can be obtained. Therefore, as an $\alpha$ amino acid used for a raw material of the silylated amino acid, a hydrophilic amino acid is preferred.

In both when the continuous phase is an aqueous substance and the dispersed phase is an oily substance, and when the continuous phase is an oily substance and the dispersed phase is an aqueous substance, a microcapsule containing core material of the present invention can be formed, although the microcapsule containing therein an oily material will have stronger capsule wall and cause leaching of core material much less than the microcapsule containing therein an aqueous material. Thus, as a preferred embodiment of the second embodiment, a microcapsule containing core material in which the continuous phase is an aqueous substance and the dispersed phase is an oily substance is provided (claim 7).

The present invention provides a cosmetic characterized in that the microcapsules containing core material of the first embodiment or the second embodiment are contained (claim 9) as the third embodiment. By blending the microcapsules containing core material of the first embodiment or the second embodiment in the cosmetic, the core material in the microcapsules can exert its effect on hair or skin and the like.

Although preferred range of content of microcapsules containing core material of the first embodiment or the

second embodiment in a cosmetic of the third embodiment varies depending on the type and form of cosmetic and the type of core material, the range of 0.01 mass% to 35 mass% in cosmetic is often preferred to make the core material exert its effect sufficiently. Thus, as a preferred embodiment of the third embodiment, a cosmetic containing 0.01 mass% to 35 mass% of the microcapsules containing core material described above is provided (claim 10).

EFFCT OF THE INVENTION

The microcapsule containing core material of the present invention is easy to manufacture, and has excellent properties of less leaching of core material, generating almost no odor from the wall material, having good storage stability since aggregation or precipitation when blended in cosmetics is suppressed. Moreover, since the capsule wall has high membrane strength, it is possible to reduce the thickness of the capsule wall and the effect of the core material can be exhibited sufficiently.

BRIEF DESCRIPTION OF THE DRAWINGS

[FIG. 1] The electron micrograph of the microcapsule containing core material prepared in Example 1.
[FIG. 2] The particle size distribution of the microcapsules containing core material prepared in Example 1.
[FIG. 3] The particle size distribution of the microcapsules containing core material prepared in Reference Example 1.

MODES FOR CARRYING OUT THE INVENTION

First, the silylated amino acid and the silane compound will be described which are materials used in the manufacture of the microcapsules containing core material of the first embodiment or the second embodiment.

[Silylated amino acid]

Alfa amino acids used in the preparation of the silylated amino acids are not particularly limited if they have been used in cosmetics. For example, any of acidic amino acids such as aspartic acid, glutamic acid or the like, neutral amino acids such as glycine, alanine, serine, threonine, methionine, cysteine, valine, leucine, isoleucine, phenylalanine, tyrosine, proline, hydroxyproline, tryptophan, asparagine, glutamine or the like and basic amino acids such as arginine, lysine, histidine, ornithine or the like can be used.

As the $\alpha$ amino acid constituting the silylated amino acid, as described above, hydrophilic amino acids are preferred. Therefore, silylated amino acids having both a hydrophobic portion and a hydrophilic portion are preferred. Here, hydrophilic amino acids mean amino acids having the solubility in water at 25 °C of 10% or more. Examples of the hydrophilic amino acids include aspartic acid, glutamic acid, glycine, alanine, serine, proline and the like. Among the hydrophilic amino acids, use of neutral amino acid having no charge is preferred, since efficient encapsulation of the dispersed phase can be achieved and stronger capsules with superior stability can be obtained. That is, neutral amino acids such as serine, glycine, alanine, proline and the like are more preferred.

The silylated amino acid in which a silyl group represented by the above described general formula (III) is bonded to an amino group of an amino acid can be obtained by reacting a silane coupling agent which will generate two or more hydroxyl groups bonded to silicon atom with an amino group of an amino acid. As the silane coupling agent which will generate two or more hydroxyl groups bonded to silicon atom, for example, 3-glycidoxypropyltrimethoxysilane, 3-glycidoxypropyltriethoxysilane, 3-glycidoxypropylmethyldimethoxysilane, 3-glycidoxypropylmethyldiethoxysilane, 3-methacryloxypropyltrimethoxysilane, 3-methacryloxypropylmethyldimethoxysilane, 3-methacryloxypropyltriethoxysilane, 3-methacryloxypropylmethyldiethoxysilane, N-(2'-aminoethyl)-3-aminopropyltriethoxysilane, N-(2'-aminoethyl)-3-aminopropylmethyldiethoxysilane and the like can be mentioned. In any of the above-mentioned agents, commercially available one can be used. For example, KBM-403, KBM-402, KBE-403, KBE-402, KBM-502, KBM-503, KBE-502, KBE-503, KBM-602, KBM-603, KBE-603 (all trade names) manufactured by Shin-Etsu Chemical Co., SH6020, SZ6023, SZ6030, SH6040 (all trade names) manufactured by Toray Dow Corning Co., Ltd. and the like correspond to those commercially available.

The silylated amino acid can be produced by a production method described in JP-A-8-59424 and JP-A-8-67608. Specifically, first, a silane coupling agent having two or more hydroxyl groups bonded to silicon atom is produced. The silane coupling agent having two or more hydroxyl groups bonded to silicon atom, thus produced, is added dropwise to an $\alpha$ amino acid solution with stirring and warming, under a basic condition of pH9-11, to make them to contact each other. Thereby the silane coupling agent is bonded to the amino group of the $\alpha$ amino acid, and the amino acid is obtained which has a silyl functional group having two or more hydroxyl groups bonded to silicon atom as shown in the general

formula (III).

A silane coupling agent having two or more of hydroxyl groups bonded to silicon atom can be prepared, for example, by stirring an acidic or basic aqueous solution of a silane coupling agent having alkoxy groups bonded to silicon atom at 30-50 °C for 5-20 min to convert the alkoxy groups bonded to the silicon atom to hydroxyl groups. When a silane coupling agent having alkoxy groups bonded to silicon atom is added dropwise into solution in a range of pH 9-11 to react with an amino acid, the alkoxy groups are hydrolyzed and converted to hydroxyl groups. That is, it is not necessary to hydrolyze the silane coupling agent having alkoxy groups in advance, and the reaction can be carried out directly by adding the silane coupling agent having alkoxy groups to an aqueous amino acid solution in which the pH is adjusted to 9-11.

The amino acid used in the reaction may be one kind of amino acid or a mixture of two or more amino acids. However, reactivities with the silane coupling agent are different depending on the amino acids, and when using a mixture of amino acids, the extents of the coupling reaction producing silylated amino acids which will be used for the construction of capsule wall, will decrease. Therefore, for producing the mixture of two or more kinds of silylated amino acid, it is desirable to prepare each silylated amino acid independently and mix them when the preparation of microcapsules is conducted.

Progress and end of the reaction can be confirmed by measuring the amino nitrogen content in the reaction solution by Van Slyke method. After completion of the reaction, the concentration is adjusted and then, the reaction product is subjected to the following capsule wall formation reaction of microcapsules with a silane compound. Alternatively, the reaction mixture obtained as described above can be neutralized, suitably concentrated, treated with ion-exchange resin, subjected to dialysis, electrodialysis and/or ultrafiltration, and the like to remove unreacted substances and impurities, then used for the starting material of the co-polycondensation with silane compound.

[Silane compound used for forming the capsule wall of the microcapsule]

Next, the silylated amino acid obtained as described above is reacted with one or more silane compounds represented by the general formula (IV) to prepare a prepolymer for the capsule and form the capsule wall of the microcapsule. Silane compound represented by the general formula (IV) can be produced by hydrolysis of a silane compound represented by the following general formula (V):

$$R^{21}{}_{p}SiX_{(4-p)} \qquad (V)$$

, wherein, p is an integer from 0 to 2, $R^{21}$ is an alkyl group having 1 to 20 carbon atoms or a phenyl group, p of $R^{21}$ may be the same or different, (4-p) of X are selected from the group consisting of a hydrogen atom, hydroxyl group, alkoxy groups having 1 to 6 carbon atoms, halogen groups, carboxy groups and amino groups.

Examples of the silane compounds represented by formula (V) include; tetramethoxysilane, methyltrimethoxysilane, ethyltrimethoxysilane, dimethyldimethoxysilane, phenyltrimethoxysilane, diphenyldimethoxysilane, n-propyltrimethoxysilane, diisopropyldimethoxysilane, isobutyltrimethoxysilane, diisobutyldimethoxysilane, hexyltrimethoxysilane, decyltrimethoxysilane, octadecyltrimethoxysilane, phenyltrimethoxysilane, tetraethoxysilane, methyltriethoxysilane, dimethyldiethoxysilane, octyltriethoxysilane, phenyl triethoxysilane, diphenyldiethoxysilane, hexyltriethoxysilane, methyltrichlorosilane, dimethyldichlorosilane, phenyltrichlorosilane, diphenyldichlorosilane, and the like.

The silane compound represented by the general formula (V) may be a commercially available product. Examples of the commercially available product include; KBM-22, KBM-103, KBE-13, KBE-22, KBE-103, KA-12, KA-13, KA-103, KA-202 (all trade names) manufactured by Shin-Etsu Chemical Co., Ltd., Z-6366, Z-6329, Z-6124, Z-6265, Z-6258, Z-2306, Z-6275, Z-6403, Z-6124, Z- 6586, Z-6341, Z-6586, ACS-8 (all trade names) manufactured by Toray Dow Corning Co., Ltd. and the like.

Next, the continuous phase and the dispersed phase where co-polycondensation of the silylated amino acid and the silane compound is performed, and the production method of microcapsules containing core material will be described.

[Continuous phase and Dispersed phase]

In the manufacture of microcapsules containing core material, the dispersion, where co-polycondensation of the silylated amino acid and the silane compound represented by the general formula (V) is performed, is prepared by dispersing a dispersed phase in a continuous phase.

An aqueous solvent may be used as the continuous phase and an oily substance may be used as the dispersed phase, as well as an oily substance may be used as the continuous phase and an aqueous material may be used as the dispersed phase. In either case, the manufacture of the capsule is possible, and microcapsules containing the dispersed phase as core material can be obtained. However, microcapsules containing an oily substance which is

produced by using an aqueous solvent as the continuous phase and an oily substance as the dispersed phase can be microcapsules having stronger capsule wall and causing almost no leaching of core material, therefore, are more preferred.

Examples of material which can be used as the core material of microcapsule and used in the dispersion phase include;

water, aqueous solutions of water-soluble substance, higher fatty acids, hydrocarbons, organic solvents, esters, phenols, silicones, silanes, metal alkoxides, higher alcohols, animal and vegetable oils, animal and plant extracts, electron-donating coloring organic compounds, dyes, ultraviolet absorbers, vitamins, medicinal ingredients, flavor components, salts, amino acids, proteins, sugars, enzymes, fluorocarbon materials, and the like. The core material can be one of above substances or mixture of two or more of them.

[Production of the microcapsule containing core material]

Co-polycondensation of a silylated amino acid and a silane compound represented by the general formula (V) can be carried out according to the production method of the microcapsules having capsule wall made of a copolymer of silylated peptide and silane compound described in Patent Document 3 and Patent Document 4. That is, when the core material (the contained material) is an oily substance, firstly, a prepolymer is prepared by reacting a silane compound represented by the general formula (IV), which is a hydrolyzate of a silane compound represented by the general formula (V), with a silylated amino acid in an aqueous solution. In the reaction, the silane compound represented by the general formula (V) is hydrolyzed in advance in an acidic or a basic solution to form the silane compound represented by the general formula (IV), followed by a reaction with the silylated amino acid. However, the reaction of the silane compound represented by the general formula (IV) with the silylated amino acid is usually carried out under acidic conditions of pH4 or lower pH or basic conditions of pH9 or higher pH, and, under the pH conditions, the silane compound represented by the general formula (V) is hydrolyzed to the silane compound represented by the general formula (IV). Therefore, hydrolyzation of the silane compound represented by general formula (V) in advance to obtain the silane compound represented by the general formula (IV) is not necessary.

Specifically, although, when the core material is an oily substance, the conditions are not particularly limited, the prepolymer can be obtained by the reaction described next.

The aqueous solution of silylated amino acid is adjusted to pH1-5, preferably pH2-4,

the silane compound represented by the general formula (V) is added dropwise to the solution at a temperature of -5-90 °C, preferably 5-75 °C, more preferably 40-60 °C, with stirring at 100-400rpm, preferably 200-300rpm,
after completion of the addition, the reaction is continued with stirring at 40-60 °C, and then
pH of the solution is adjusted to about 5-7. As the acidic agent for pH adjustment in the manufacture, for example, inorganic acids such as hydrochloric acid, sulfuric acid, phosphoric acid or the like; organic acids such as acetic acid or the like can be used. As the alkali agent, for example, sodium hydroxide, potassium hydroxide or the like can be used.

Next, with stirring the dispersion containing prepolymer prepared as described above at 500-700 rpm, preferably at about 550-650rpm, a phase to become the core material is added over 30 minutes to 3 hours at 30-70 °C, preferably at 45-55 °C. After completion of the addition, the dispersion is stirred more for 2-5 hours by a homomixer at 5,000-15,000 rpm, preferably 8,000 ~ 12,000 rpm to fully perform emulsification thereby a capsule wall is formed.

When the core material is an aqueous substance, after preparation of the prepolymer, a large amount of oily substance is added, with stirring at 500-700 rpm, preferably at about 600 rpm, to reverse the phase and microcapsules containing an aqueous substance as the core material can be obtained.

Thereafter, distillation operation of generated alcohol and pH adjustment are carried out and microcapsules containing core material can be obtained. After the microcapsules containing core material are obtained, they may be treated further with the silane compound represented by the above described general formula (V), for strengthening the capsule wall (capsule wall strengthening treatment). However, when the core material is a light sensitive agent, such as ultraviolet absorber, there is a possibility that activity of ultraviolet absorption is reduced if the capsule wall is too thick. In addition, there is a possibility to feel the foreign body sensation when applied on skin if the capsule wall becomes too thick and the capsules become hard. Therefore, the extent of the capsule wall strengthening treatment must be determined properly based on the usage of the microcapsule containing core material.

Although it is possible to obtain microcapsules containing material as described above, unreacted hydroxyl groups may remain in the co-polycondensate constituting the capsule wall. If hydroxyl groups remain, the hydroxy groups on the capsule surface may bond to each other which may cause aggregation and precipitation of the capsules. Therefore, it is preferable to carry out surface treatment of capsules to prevent aggregation of the microcapsules containing core material obtained as described above.

The surface treatment of capsules is carried out by using a silane compound having one hydroxyl group bonded to silicon atom represented by the following general formula (VI):

$R^4{}_3SiOH$ (VI)

, wherein $R^4$ is an alkyl group having 1 to 4 carbon atoms or a phenyl group, the three alkyl groups may be the same or different each other, to block the hydroxyl groups in the co-polycondensate constituting the capsule wall.

The silane compound having one hydroxyl group represented by the general formula (VI) can be obtained by hydrolysis of a silane compound represented by the general formula (VII):

$R^4{}_3Si\text{-}R^5$ (VII)

, wherein, $R^4$ is an alkyl group having 1 to 4 carbon atoms or a phenyl group, the three alkyl groups may be the same or different each other, $R^5$ is an alkoxy group having 1 to 6 carbon atoms or a halogen atom.

Examples of the silane compound represented by the general formula (VII) include; trimethylsilylchloride(trimethylchlorosilane), triethylsilylchloride(triethylchlorosilane), t-butyldimethylsilylchloride(t- butyldimethylchloro silane), triisopropylsilylchloride (triisopropyl chlorosilane), trimethylethoxysilane, triphenylethoxysilane and the like. Commercially available products can be used as the above compound. As commercially available products, for example, LS-260, LS-1210, LS-1190, TIPSC (all trade names) manufactured by Shin-Etsu Chemical Co., Ltd., Z-6013 (trade name) manufactured by Toray Dow Corning Co., Ltd. and the like can be mentioned.

The surface treatment such as capsule wall strengthening treatment and treatment for preventing aggregation of the capsules can be carried out either by adding the silane compound, in which a hydroxyl group was generated by hydrolysis in advance, to the solution containing the capsules, or by adjusting pH of the solution to a pH where the silane compound to be used for the treatment hydrolyzes, followed by adding the silane compound to the solution. That is, the treatment can be carried out by adjusting pH of the solution containing the capsules to pH 2-4, followed by adding a silane compound represented by the general formula (VII) to the solution with stirring the solution preferably in the range of

40-75 °C at 300-800 rpm. After completion of the addition, the reaction is continued for an additional 2-5 hours with stirring for sufficient progress of the reaction.

Next, cosmetics containing the above described microcapsules containing core material of the present invention (the first embodiment, the second embodiment) will be described.

[Cosmetics comprising microcapsules containing core material]

A cosmetic of the present invention is prepared by compounding microcapsules containing core material having capsule wall made of a silylated amino acid/silane compound copolymer manufactured as mentioned above. As examples of the cosmetic, skin cosmetics such as skin creams, milky lotions, cleansing liquids, cleansing creams, skin care gels, essences, sunscreen creams, and the like, and hair cosmetics such as hair rinses, hair treatments, hair conditioners, hair creams, split hair coating agents, shampoos, hair setting agents, hair dyes, agents for a permanent wave, and the like can be mentioned.

Content of the microcapsule containing core material of the present invention (the first embodiment, the second embodiment) in the cosmetic of the present invention (amount in the cosmetic) varies depending on the type of the cosmetic, but, in many cases, the content is preferably 0.01% by mass - 35% by mass, and more preferably 1 % by mass - 20 % by mass. When content of the microcapsule containing core material is less than the above range, it may not be possible to exhibit the effect of the core material. When content of the microcapsule containing core material in the cosmetic is more than the above range, stickiness, roughness or foreign body sensation occurs sometimes.

The cosmetic of the present invention contains microcapsules containing core material of the present invention as an essential component, and may contain anionic surfactants, nonionic surfactants, cationic surfactants, amphoteric surfactants, cationic polymers, amphoteric polymers, anionic polymers, thickeners, animal and plant extracts, polysaccharides or derivatives thereof, hydrolysates of the proteins from animals, plants or microorganisms and derivatives thereof, a neutral or acidic amino acids, wetting agents, lower alcohols, higher alcohols, fats and oils, silicones, various dyes and pigments, preservatives, perfumes, and the like, as long as the gist of the present invention is not impaired.

EXAMPLES

Then, the present invention will be illustrated with reference to examples, but the scope of the present invention is not limited to these examples. Prior to the examples, production examples of silylated amino acids used in Examples are described. Any "%" described in the following examples and production examples are "mass%".

Production Example 1: Production of N-[2-hydroxy-3-[3'-(trihydroxysilyl)propoxy]propyl]serine (silylated serine)

Into a 1 liter beaker, 30g (0.285 mol) of serine and, then, 270g of water were added. The resulting mixture was stirred and adjusted to pH9.2 by addition of 25% aqueous sodium hydroxide.
The resulting solution was warmed to 50 °C. To the warmed solution, 3-glycidoxypropyltriethoxysilane [KBE-403 (trade name); manufactured by Shin-Etsu Chemical Co., Ltd.] 79.5g (0.286 mol; equimolar amounts of serine) was added dropwise over about 2 hours with stirring and stirring was continued for 16 hours at 50 °C after completion of the addition. Thereafter, 17% aqueous hydrochloric acid was added to adjust to pH6.0 and the solution was stirred for 1 hour at 80 °C and then cooled to obtain 403.9g of an aqueous solution of N-[2-hydroxy-3-(3'-trihydroxysilyl)propoxy] propyl]seri ne (silylated serine) having the solid concentration of 18.2%. The extent of the reaction calculated based on the amino nitrogen contents before and after the reaction was 75.6%. As the result, moles of N-[2-hydroxy-3-[3'-(trihydroxysilyl)propoxy]propyl]serine was calculated and determined to be 0.186.
The reaction solutions before and after the reaction were analyzed by liquid chromatography under the following conditions (HPLC, hereinafter referred to as HPLC) . After the reaction, the peak in the vicinity of molecular weight 105 of the raw material of serine almost disappeared, and a new peak near 298, the molecular weight of N-[2-hydroxy-3-[3'-(trihydroxysilyl)propoxy]propyl] serine, was detected. Thus, it was confirmed that N-[2-hydroxy-3-[3'-(trihydroxysilyl)propoxy]propyl] serine was produced.

[Analysis conditions of liquid chromatography (HPLC)]

Separation column: TSKgel G3000PWxL (diameter 7.8 mm × length 300 mm)
Eluent: 0.1% aqueous trifluoroacetic acid / acetonitrile = 55/45
Flow rate: 0.3mL/min
Detector: RI (differential refractive index) detector and UV (ultraviolet) detector (210 nm)
Standard samples: glutathione (Mw307), bradykinin
(Mw1, 060), insulin B chain (Mw3, 496), aprotinin (Mw6, 500)

Production Example 2: Production of N-[2-hydroxy-3-[3'-(trihydroxysilyl)propoxy]propyl]glycine

In the same manner as in production example 1 except for using glycine 30g (0.4 mol) instead of serine and using 3-glycidoxypropyl triethoxysilane 111.2g (0.4 mol, equimolar amounts of glycine), 432.7g of an aqueous solution of N-[2-hydroxy-3-[3'-(trihydroxysilyl) propoxy]propyl]glycine (silylated glycine) having the solid concentration of 22.9% was obtained. The extent of the reaction calculated based on the amino nitrogen contents before and after the reaction was 62.2%. As the result, moles of N-[2-hydroxy-3-[3'-(trihydroxysilyl) propoxy]propyl]glycine was calculated and determined to be 0.23.
As a result of HPLC analysis under the same conditions as in production example 1, the peak of molecular weight of about 75 of glycine prior to reaction disappeared after the reaction, a peak was detected around 268 and it was confirmed that N-[2-hydroxy-3-[3'-(trihydroxysilyl) propoxy]propyl]glycine was produced.

Production Example 3: Production of N-[2-hydroxy-3 -[3'-(trihydroxysilyl)propoxy]propyl]aspartic acid (silylated aspartic acid)

In the same manner as in production example 1 except for using aspartic acid 30g (0.225 mol) instead of serine and using 3-glycidoxypropyl triethoxysilane 55.8g (0.225 mol, equimolar amounts of aspartic acid), 365.8g of solution of N-[2-hydroxy-3-[3'-(trihydroxysilyl) propoxy]propyl]aspartic acid (silylated aspartic acid) having the solid concentration of 17.4% was obtained. The extent of the reaction calculated based on the amino nitrogen contents before and after the reaction was 78%. As the result, moles of N-[2-hydroxy-3-[3'-(trihydroxysilyl)propoxy]propyl]aspartic acid was calculated and determined to be 0.15.
As a result of HPLC analysis under the same conditions as in production example 1, the peak of molecular weight of about 133 of aspartic acid prior to reaction disappeared after the reaction, a new peak was detected around 330 and it was confirmed that N-[2-hydroxy-3-[3'-(trihydroxysilyl)propoxy]propyl]aspartic acid was produced.

Production Example 4: Production of N-[2-hydroxy-3-[3'-(trihydroxysilyl)propoxy]propyl]glutamic acid (silylated glutamic acid)

In the same manner as in production example 1 except for using glutamic acid 50g (0.340 mol) instead of serine

and using 3-glycidoxypropyl triethoxysilane 94.6g (0.340 mol, equimolar amounts of glutamic acid), 1070.9g of a solution of N-[2-hydroxy-3-[3'-(trihydroxy silyl)propoxy]propyl]glutamic acid (silylated glutamic acid) having the solid concentration of 11.4% was obtained. The extent of the reaction calculated based on the amino nitrogen contents before and after the reaction was 63.3%. As the result, moles of N-[2-hydroxy-3-[3'-(trihydroxysilyl)propoxy]propyl]glutamic acid was calculated and determined to be 0.28.

As a result of HPLC analysis under the same conditions as in production example 1, the peak of molecular weight of about 147 of glutamic acid prior to reaction disappeared after the reaction, a new peak was detected around 340 and it was confirmed that N-[2-hydroxy3-[3'-(trihydroxysilyl)propoxy]propyl]glutamic acid was produced.

Production Example 5: Production of N-[2-hydroxy-3-[3'-(trihydroxysilyl)propoxy]propyl]lysine (silylated lysine)

In the same manner as in production example 1 except for using lysine hydrochloride 30g (0.164 mol) instead of serine in production example 1 and using 3-glycidoxypropyl triethoxysilane 45.7g (0.164 mol, equimolar amounts of lysine), 378.0g of solution of N-[2-hydroxy-3-(3'-trihydroxysilyl)propoxy]propyl]lysine (silylated lysine) having the solid concentration of 13.5% was obtained. The extent of the reaction calculated based on the amino nitrogen contents before and after the reaction was 42.0%. Lysine has two amino groups, and it seemed that 80% or more of silyl groups was introduced to the amino group at $\alpha$-position. As the result, moles of N-[2-hydroxy-3-[3'-(trihydroxysilyl)propoxy]propyl] lysine was calculated and determined to be 0.13.

As a result of HPLC analysis under the same conditions as in production example 1, the peak of molecular weight of about 146 of lysine prior to reaction disappeared after the reaction, peaks were detected near the vicinity of 340 and 530, and it was confirmed that N-[2-hydroxy-3-(3'-trihydroxysilyl)propoxy]propyl]lysine or N,N'-di-[2-hydroxy-3-(3'-trihydroxysilyl)propoxy]propyl]lysine was produced.

Production Example 6: Production of N-[2-hydroxy-3-[3'-(trihydroxysilyl)propoxy]propyl] arginine (silylated arginine)

In the same manner as in production example 1 except for using arginine 40 g (0.23 mol) instead of serine in production example 1 and using 3-glycidoxypropyl triethoxysilane 76.7g (0.275 mol, 1.2 molar equivalent of arginine), 274.9g of an aqueous solution of N-[2-hydroxy-3-(3'-trihydroxysilyl)propoxy]propyl] arginine(silylated arginine) having the solid concentration 25.8 % was obtained. The extent of the reaction calculated based on the amino nitrogen contents before and after the reaction was 85.9%. As the result, moles of N-[2- hydroxy-3-[3'-(trihydroxysilyl)propoxy] propyl] arginine was calculated and determined to be 0.16.

As a result of HPLC analysis under the same conditions as in production example 1, the peak of molecular weight of about 174 of arginine prior to reaction disappeared after the reaction, a peak was detected near the vicinity of 370, and it was confirmed that N-[2-hydroxy-3-(3'-trihydroxysilyl) propoxy]propyl]arginine was produced.

Production Example 7: Production of N-[2-hydroxy-3-[3'-(trihydroxysilyl)propoxy]propyl]alanine (silylated alanine)

In the same manner as in production example 1 except for using alanine 30g (0.337 mol) instead of serine in production example 1 and using 3-glycidoxypropyl triethoxysilane 93.8g (0.337 mol, equimolar amounts of alanine), 317.7g of a solution of N-[2-hydroxy-3-[3'-(trihydroxysilyl)propoxy]propyl]alanine (silylated alanine) having the solid concentration of 25.3% was obtained. The extent of the reaction calculated based on the amino nitrogen contents before and after the reaction was 70.3%. As the result, moles of N-[2-hydroxy-3-[3'-(trihydroxysilyl)propoxy]propyl]alanine was calculated and determined to be 0.2.

As a result of HPLC analysis under the same conditions as in production example 1, the peak of molecular weight of about 89 of alanine prior to reaction disappeared after the reaction, a new peak was detected near the vicinity of 280, and it was confirmed that N-[2-hydroxy-3-[3'-(trihydroxysilyl)propoxy]propyl]alanine was produced.

Production Example 8: Production of N-[2-hydroxy-3-[3' -(dihydroxymethylsilyl)propoxy]propyl]serine (silylated serine)

Into a 1 liter beaker, 30g (0.285 mol) of serine and, then, 270g of water were added. The resulting mixture was stirred and adjusted to pH9.2 by addition of 25% aqueous sodium hydroxide. The resulting solution was warmed to 60 °C. To the warmed solution, 3-glycidoxypropylmethyldiethoxysilane [KBE-402 (trade name); manufactured by Shin-Etsu Chemical Co., Ltd.] 70.7g (0.285 mol, equimolar amounts of serine) was added dropwise over about 2 hours with stirring and stirring was continued for 16 hours at 60 °C after the dropwise addition. Thereafter, 17% aqueous hydrochloric acid was added to adjust to pH6.0 and the solution was stirred for 1 hour at 80 °C and then cooled to obtain 458.9g of an aqueous solution of N-[2-hydroxy-3-(3'-dihydroxymethylsilyl) propoxy]propyl]serine (silylated serine) having the solid concentration of 18.4%. The extent of the reaction calculated based on the amino nitrogen contents before and after the reaction was 77%. As the result, moles of N-[2-hydroxy-3-[3'-(dihydroxymethylsilyl)propoxy] propyl]serine was calculated and determined to be 0.22.

Example 1: Production of microcapsules having capsule wall made of a co-polycondensate of silylated serine and methyltriethoxysilane and containing an ultraviolet absorber

Into a 2 liter glass lid circular reactor, 131.9g of 18.2% aqueous solution of silylated serine (N-[2-hydroxy -3-[3'-(trihydroxysilyl)propoxy]propyl]serine) (about 0.061 mole of silylated serine) prepared in production example 1 was charged. Then, 68.1g of water was added to adjust the solid concentration to 12%, and 17% aqueous hydrochloric acid was added to adjust to pH2.2.

The resulting solution was warmed to 50 °C and to the solution, methyltriethoxysilane [KBE-13 (trade name); manufactured by Shin-Etsu Chemical Co. , Ltd.] 33.5 g (0.188 mol) was added dropwise with stirring over about 30 minutes. After the completion of the addition, stirring was continued for 4 hours at 50 °C. Then, aqueous sodium hydroxide was added dropwise to adjust pH of the solution to 6.0, and thereto, 2-ethylhexyl p-methoxycinnamate 345.1g was added dropwise over 2.5 hours with stirring at 600 rpm. Thereafter, the solution was stirred at 10,000 rpm using a homomixer at 50 °C, and finely emulsified.

Next, for the surface treatment of capsule wall for preventing aggregation, trimethylchlorosilane [LS-260 (trade name) manufactured by Shin-Etsu Chemical Co., Ltd.] 5.1 g was added to the resulting emulsion with stirring at 400 rpm at 50 °C, the pH was adjusted to 6.0 with 5% aqueous sodium hydroxide, and the resulting reaction solution was heated to reflux. After distilling off the vapor containing alcohol, the reflux was further continued for 2 hours with stirring at 400 rpm.

The resulting reaction solution was slowly cooled to room temperature with stirring at 100 rpm and 645.7 g of dispersion of microcapsule (solid concentration 60.6%) containing 2-ethylhexyl p-methoxycinnamate (ultraviolet absorber) and having capsule wall made of a co-polycondensate of silylated serine and methyltriethoxysilane was obtained.

The resulting capsule dispersion was observed with electron microscope, and it was confirmed that microcapsules having the particle size of about 2 μm were generated, as shown in FIG. 1. Further, the resulting microcapsule dispersion was measured by particle size distribution analyzer described below, and it was confirmed that the capsules have an average particle diameter of 1.553 μm with a narrow distribution range of standard deviation of 0.229 as shown in FIG. 2.

Example 2: Production of microcapsules having capsule wall made of a co-polycondensate of silylated glycine and methyltriethoxysilane and containing an ultraviolet absorber

Into a 2 liter glass lid circular reactor, 78.6g of 22.9% aqueous solution of silylated glycine (N-[2-hydroxy -3-[3'-trihydroxysilyl)propoxy]propyl]glycine) (about 0.042 mole of silylated glycine) prepared in production example 2 was charged. Then, 221.4g of water was added to adjust the solid concentration to 6%, and 17% aqueous hydrochloric acid was added to adjust to pH2.2.

The resulting solution was warmed to 50 °C and to the solution, methyltriethoxysilane 36.3g (0.202 mol) was added dropwise with stirring over about 30 minutes. After the completion of the dropwise addition, stirring was continued for 4 hours at 50 °C.

Then, an aqueous sodium hydroxide was added dropwise to adjust the pH of the solution to 6.0, and thereto, 2-ethylhexyl p-methoxycinnamate 362.7g was added dropwise over 2.5 hours with stirring at 600 rpm. Thereafter, the solution was stirred at 10,000 rpm using a homomixer at 50 °C, and finely emulsified.

With stirring the resulting emulsion at 400 rpm at 50 °C, trimethylchlorosilane 5.5 g was added for the surface treatment of capsule wall for preventing aggregation, pH was adjusted to 6.0 with 5% aqueous sodium hydroxide, and the resulting reaction solution was heated to reflux. After distilling off the vapor containing alcohol, the reflux was further continued for 2 hours with stirring at 400 rpm.

The resulting reaction solution was slowly cooled to room temperature with stirring at 100 rpm and 683 g of dispersion of microcapsules (solid concentration 58.3%) containing 2-ethylhexyl p-methoxycinnamate and having capsule wall made of a co-polycondensate of silylated glycine and methyltriethoxysilane was obtained.

Example 3: Production of microcapsules having capsule wall made of a co-polycondensate of silylated aspartic acid and methyltriethoxysilane and containing an ultraviolet absorber

In the same manner as in example 1 except for using 138g of 17.4% aqueous solution of silylated aspartic acid (N-[2-hydroxy-3-[3'-(trihydroxysilyl) propoxy]propyl]aspartic acid) produced in production example 3 as the raw material silylated amino acid, changing the amounts of water for adjusting the concentration to 62.0g, of methyltriethoxysilane to 41.6g, and of 2-ethylhexyl p-methoxycinnamate to be contained to 270.2g, and using 3.3g of trimethylchloro silane as the silane compound for the surface treatment of capsule wall, 570.7g of dispersion of microcapsules (solid concentration 58.2%) having capsule wall made of a co-polycondensate of silylated aspartic acid and methyltriethoxysilane and containing 2-ethylhexyl p-methoxycinnamate was obtained.

Example 4: Production of microcapsules having capsule wall made of a co-polycondensate of silylated glutamic acid and methyltriethoxysilane and containing an ultraviolet absorber

In the same manner as in example 2 except for using 92.2g of 11.4% aqueous solution of silylated glutamic acid (N-[2-hydroxy-3-[3'-(trihydroxysilyl) propoxy]propyl]glutamic acid) produced in production example 4 as the raw material silylated amino acid, changing the amounts of water for adjusting the concentration to 82.8g, of methyltriethoxysilane to 44.0g and of 2-ethylhexyl p-methoxycinnamate to be contained to 324.5g, and using 2.7g of trimethylchloro silane as the silane compound for the surface treatment of capsule wall, 590g of dispersion of microcapsules (solid concentration 60.9%) having capsule wall made of a co-polycondensate of silylated glutamic acid and methyltriethoxysilane and containing 2-ethylhexyl p-methoxycinnamate was obtained.

Example 5: Production of microcapsules having capsule wall made of a co-polycondensate of silylated lysine and methyltriethoxysilane and containing an ultraviolet absorber

In the same manner as in example 2 except for using 88.8g of 13.5% aqueous solution of silylated lysine (N-[2-hydroxy-3-[3'-(trihydroxysilyl) propoxy]propyl]lysine) produced in production example 5 as the raw material silylated amino acid, changing the amounts of water for adjusting the concentration to 111.2g, of methyltriethoxysilane to 44.0g and of 2-ethylhexyl p-methoxycinnamate to be contained to 305.7g, and using 3.1g of trimethylchlorosilane as the silane compound for the surface treatment of capsule wall, 570.7g of dispersion of microcapsules (solid concentration 58.2%) having capsule wall made of a co-polycondensate of silylated lysine and methyltriethoxysilane and containing 2-ethylhexyl p-methoxycinnamate was obtained.

Example 6: Production of microcapsules having capsule wall made of a co-polycondensate of silylated arginine and methyltriethoxysilane and containing an ultraviolet absorber

In the same manner as in example 2 except for using 46.4g of 25.8% aqueous solution of silylated arginine (N-[2-hydroxy-3-[3'-(trihydroxysilyl)propoxy]propyl] arginine) produced in production example 6 as the raw material silylated amino acid, changing the amounts of water for adjusting the concentration to 153.6g, of methyltriethoxysilane to 47.3g and of 2-ethylhexyl p-methoxycinnamate to be contained to 153.6g, and using trimethylchlorosilane 2.9g and trimethylethoxysilane 9.4g as the silane compound for the surface treatment of capsule wall, 306.2g of dispersion of microcapsules (solid concentration 60.7%) having capsule wall made of a co-polycondensate of silylated arginine and methyltriethoxysilane and containing 2-ethylhexyl p-methoxycinnamate was obtained.

Example 7: Production of microcapsules having capsule wall made of a co-polycondensate of silylated alanine and methyltriethoxysilane and containing an ultraviolet absorber

In the same manner as in example 2 except for using 47.3g of 25.3% aqueous solution of silylated alanine (N-[2-hydroxy-3-[3'-(trihydroxy silyl)propoxy]propyl] alanine) produced in production example 7 as the raw material silylated amino acid, changing the amounts of water for adjusting the concentration to 152.7g, of methyltriethoxysilane to 34.9g and of 2-ethylhexyl p-methoxycinnamate to be contained to 250.1g, and using 3.5g of trimethylchlorosilane as the silane compound for the surface treatment of capsule wall, 451.9g of dispersion of microcapsules (solid concentration 60.2%) having capsule wall made of a co-polycondensate of silylated alanine and methyltriethoxysilane and containing 2-ethylhexyl p-methoxycinnamate was obtained.

Example 8: Production of microcapsules having capsule wall made of a co-polycondensate of silylated serine, silylated aspartic acid and methyltriethoxysilane and containing an ultraviolet absorber

In the same manner as in example 1 except for using a mixture of 63.8g of 18.2% aqueous solution of silylated serine produced in production example 1 and 69g of 17.4% aqueous solution of silylated aspartic acid produced in production example 3 as the raw material silylated amino acid, changing the amounts of water for adjusting the concentration to 67.2 g, of methyltriethoxy silane to 40 g and of 2-ethylhexyl p-methoxycinnamate to be contained to 306. 2g, and using 5.1g of trimethylchlorosilane as the silane compound for the surface treatment of capsule wall, 543.6g of dispersion of microcapsules (solid concentration 60.5%) having capsule wall made of a co-polycondensate of silylated serine, silylated aspartic acid and methyltriethoxysilane and containing 2-ethylhexyl p-methoxycinnamate was obtained.

Example 9: Production of microcapsules having capsule wall made of a co-polycondensate of silylated glycine, methyltriethoxysilane and phenyltriethoxysilane containing dimethylpolysiloxane

In the same manner as in example 2 except for using 78.6g of 22.9% aqueous solution of silylated glycine (N-[2-hydroxy-3-[3'-(trihydroxysilyl)propoxy]propyl] glycine) produced in production example 2 as the raw material silylated amino acid, changing the amount of water for adjusting the concentration to 223.4g, using a mixture of 37.4g (0.21 mol) of methyltriethoxysilane and 10g (0.04 mol) of phenyltriethoxysilane as the silane compound, using 448.2g of dimethylpolysiloxane [KF-96A-1000cs (trade name) manufactured by Shin-Etsu chemical Co., Ltd.] to be contained, and using 11.7g of trimethylchlorosilane as the silane compound for the surface treatment of capsule wall, 806g of dispersion of microcapsules (solid concentration 61%) having capsule wall made of a co-polycondensate of silylated glycine, methyltriethoxysilane and phenyltriethoxysilane and containing dimethylpolysiloxane was obtained.

Example 10: Production of microcapsules having capsule wall made of a co-polycondensate of silylated serine and methyltriethoxysilane containing ascorbyl tetrahexyldecanoate

Into a 2 liter glass lid circular reactor, 131.9g of 18.2% aqueous solution of silylated serine prepared in production example 1 was charged. Then, 88.1g of water was added to adjust the solid concentration to 12%, and 17% aqueous hydrochloric acid was added to adjust to pH2.2.

The resulting solution was warmed to 50 °C and to the solution, methyltriethoxysilane 33.5g was added dropwise with stirring over about 30 minutes. After the completion of the dropwise addition, stirring was continued for 4 hours at 50 °C.

Then, 25% aqueous sodium hydroxide was added dropwise to adjust the pH of the solution to 6.0, and thereto, ascorbyl tetrahexyldecanoate [Wako Pure Chemical Industries, Ltd.] 218.9g was added dropwise over 2.5 hours with stirring at 600 rpm.

Thereafter, trimethylchlorosilane 2.7g was added thereto at 50 °C, with stirring at 400 rpm, and 5% aqueous sodium hydroxide was added to adjust to pH6.0. Under reduced pressure at 40 °C, 104.5g of the solvent of the reaction solution was distilled off with a rotary evaporator, and 313.5g of dispersion of the microcapsules (solid concentration 87.9%) having capsule wall made of a co-polycondensate of silylated serine and methyltriethoxysilane and containing ascorbyl tetrahexyldecanoate was obtained.

Example 11: Production of microcapsules having capsule wall made of a co-polycondensate of silylated aspartic acid, methyltriethoxysilane and phenyltriethoxysilane containing squalane

Into a 2 liter glass lid circular reactor, 57.5g of 17.4% aqueous solution of silylated aspartic acid (about 0.02 mol of silylated aspartic acid) prepared in production example 3 was charged. Then, 42.5g of water was added to adjust the solid concentration to 12%, and 17% aqueous hydrochloric acid was added to adjust to pH2.2.

The resulting solution was warmed to 50 °C and to the solution, a mixture of methyltriethoxysilane 23.1g (0.133 mol) and phenyltriethoxysilane 16g (0.066 mol) was added dropwise with stirring over about 30 minutes. After the completion of the dropwise addition, stirring was continued for 4 hours at 50 °C. Then, an aqueous sodium hydroxide was added dropwise to adjust pH of the solution to 6.0, and thereto, squalane 240g was added dropwise over 1 hour with stirring at 600 rpm. Thereafter, the solution was stirred at 10,000 rpm using a homomixer at 50 °C, and finely emulsified.

Next, with stirring the resulting emulsion at 400 rpm at 50 °C, trimethylchlorosilane 5.1 g was added for the surface treatment of capsule wall, pH was adjusted to 6.0 with 5% aqueous sodium hydroxide, and the resulting reaction solution was heated to reflux. After distilling off the vapor containing alcohol, the reflux was further continued for 2 hours with stirring at 400 rpm.

The resulting reaction solution was slowly cooled to room temperature with stirring at 100 rpm and 430g of dispersion of microcapsules (solid concentration 88.1 %) containing squalane with solid concentration of 60% and having capsule wall made of a co-polycondensate of silylated aspartic acid, methyl triethoxysilane and phenyl triethoxysilane was obtained.

Example 12: Production of microcapsules having capsule wall made of a co-polycondensate of silylated serine and methyltriethoxysilane and octyltriethoxysilane containing water

Into a 2 liter glass lid circular reactor, 74.8g of 24.5% aqueous solution of N-[2-hydroxy-3-[3'-(dihydroxymethylsilyl)propoxy]propyl]serine (about 0.06 mol as silylated serine) prepared in production example 9 was charged. Then, 47.4g of water was added to adjust the solid concentration to 15%, and 17% aqueous hydrochloric acid was added to adjust to pH2.0.

The resulting solution was warmed to 50 °C and to the solution, a mixture of methyltriethoxy silane 42.7g (0.24 mol) and octyltriethoxysilane 132.5g (0.48 mol) was added dropwise with stirring over about 30 minutes. After the completion of the addition, stirring was continued for 16 hours at 50 °C.

Then, the stirring speed was increased to 600 rpm, caprylic/capric Triglyceride 259g to be the continuous phase was added, stirring was continued until the phase inversion occurred, then 20% aqueous sodium hydroxide was added to adjust to pH6, and stirring was further continued at 50 °C for 3 hours at 600 rpm.

Next, a mixture of trimethylchlorosilane 6.5g (0.06 mol) and trimethylethoxysilane 28.3g (0.24 mol) was added with stirring at 600rpm, 50 °C, and then stirring was continued for 16 hours at 600rpm, 50 °C. To the resulting reaction solution, 5% aqueous sodium hydroxide was added dropwise to adjust pH of the solution to 6. The stirring speed was decreased to 400 rpm and the reaction solution was gradually heated to 80 °C and kept under reflux for 2 hours to remove generated alcohol and water present in the continuous phase. Then, with stirring at 400 rpm, the temperature of the reaction solution was lowered to room temperature, and 443g of dispersion of microcapsules with about 40% water content in caprylic/capric Triglyceride was obtained (calculated content of capsules was 50%).

Next, Reference examples are shown which are production examples of microcapsules having capsule wall made by co-polycondensation of a silylated peptide and a silane compound, to be used as comparative products or comparative examples in the performance evaluation tests and in examples.

Since protein hydrolyzate (hydrolysed peptide) constituting the peptide portion of the silylated peptide is a mixture of peptides of different molecular weights, measured value of the amino acid polymerization degree is the average polymerization degree of the peptide (average polymerization degree of amino acid).

Reference Example 1: Production of microcapsules having capsule wall made of co-polycondensate of silylated hydrolyzed casein and methyltriethoxysilane and containing an ultraviolet absorber

Into a 1 liter beaker, 25% aqueous solution of casein hydrolyzate (hydrolysed casein) 100g (0.04 mol as moles calculated from the amino nitrogen content) having average polymerization degree of amino acid of 6 determined from the total nitrogen content and the amino nitrogen content was charged and the pH was adjusted to 9.5 by addition of 25% aqueous sodium hydroxide. The resulting solution was warmed to 50 °C. To the warmed solution, 3-glycidoxypropyltriethoxysilane 10g (0.04 mol, equimolar amounts of the amino nitrogen content of hydrolyzed casein) was added dropwise over about 1 hour with stirring. After completion of the dropwise addition, stirring was continued for 14 hours at 50 °C. Thereafter, 17% aqueous hydrochloric acid was added to adjust the pH to 6.0 and 113g of an aqueous solution of N-[2-hydroxy-3-(3'-trihydroxysilyl)propoxy]propyl] hydrolyzed casein (silylated hydrolyzed casein) having the solid concentration of 28.8% was obtained.

The extent of the reaction calculated based on the amino nitrogen contents before and after the reaction was 81%. As the result, moles of N-[2-hydroxy-3-[3'-(trihydroxysilyl)propoxy]propyl]hydrolyzed casein was calculated and determined to be 0.03.

Next, the aqueous solution of silylated hydrolyzed casein, thus prepared, was charged into a 2 liter glass lid circular reactor. Then, 158g of water was added to adjust the solid concentration to 12%, and 17% aqueous hydrochloric acid was added to adjust to pH2.2.

The resulting solution was warmed to 50 °C and to the solution, methyltriethoxysilane 21.3g (0.12 mol) was added dropwise with stirring over about 30 minutes. After the completion of the dropwise addition, stirring was continued for 4 hours at 50 °C.

Then, aqueous sodium hydroxide was added dropwise to adjust pH of the solution to 6.0, and thereto, 2-ethylhexyl p-methoxycinnamate 394g was added dropwise over 2.5 hours with stirring at 600 rpm. Thereafter, the solution was stirred at 10,000 rpm using a homomixer at 50 °C, and finely emulsified.

Next, for the surface treatment of capsule wall, trimethylchlorosilane 9.7g was added to the resulting emulsion with stirring at 400 rpm, 50 °C, pH was adjusted to 6.0 with 5% aqueous sodium hydroxide, and the resulting reaction solution was heated to reflux. After distilling off the vapor containing alcohol, the reflux was further continued for 2 hours with stirring at 400 rpm.

The resulting reaction solution was slowly cooled to room temperature with stirring at 100 rpm and solid concentration was adjusted to 60% by adding water to obtain 617g of aqueous dispersion of microcapsules containing 2-ethylhexyl p-methoxycinnamate and having capsule wall made of a co-polycondensate of silylated hydrolyzed casein and methyltriethoxysilane.

Reference Example 2: Production of microcapsules having capsule wall made of a co-polycondensate of silylated hydrolyzed pea protein, methyltriethoxysilane and phenyltriethoxysilane and containing dimethylpolysiloxane

Into a 1 liter beaker, 25% aqueous solution of pea protein hydrolyzate (hydrolysed pea protein) 100g (0.05 mol as moles calculated from the amino nitrogen content) having average polymerization degree of amino-acids of 4.5

determined from the total nitrogen content and the amino nitrogen content was charged and the pH was adjusted to 9.5 by addition of 25% aqueous sodium hydroxide.

The resulting solution was warmed to 50 °C. To the warmed solution, 3-glycidoxypropyltriethoxysilane 14g (0.05 mol, equimolar amounts of the amino nitrogen content of the hydrolyzed pea protein) was added dropwise over about 1 hour with stirring. After completion of the dropwise addition, stirring was continued for 14 hours at 50 °C. Thereafter, 17% aqueous hydrochloric acid was added to adjust to pH6.0 and 133g of an aqueous solution of N-[2-hydroxy-3-(3'-trihydroxysilyl)propoxy]propyl] hydrolyzed pea protein (silylated hydrolyzed pea protein) having the solid concentration of 26.2% was obtained.

The extent of the reaction calculated based on the amino nitrogen contents before and after the reaction was 83%. As the result, moles of N-[2-hydroxy-3-[3'-(trihydroxysilyl)propoxy]propyl]hydrolyzed pea protein was calculated and determined to be 0.04.

Next, the aqueous solution of the silylated hydrolyzed pea protein, thus prepared, was charged into a 2 liter glass lid circular reactor. Then, 157g of water was added to adjust the solid concentration to 12%, and 17% aqueous hydrochloric acid was added to adjust to pH2.2.

The resulting solution was warmed to 50 °C and to the solution, a mixture of methyltriethoxysilane 37.4g (0.21 mol) and phenyltriethoxysilane 10g (0.04 mol) was added dropwise with stirring over about 30 minutes. After the completion of the dropwise addition, stirring was continued for 4 hours at 50 °C.

Then, an aqueous sodium hydroxide was added dropwise to adjust pH of the solution to 6.0, and thereto, dimethyl polysiloxane [KF-96A-1000cs (trade name) manufactured by Shin-Etsu Chemical Co., Ltd.] 559g was added dropwise over 2.5 hours with stirring at 600 rpm. Thereafter, the solution was stirred at 10,000 rpm using a homomixer at 50 °C, and finely emulsified.

Next, for the surface treatment of capsule wall, trimethylchlorosilane 11.7g was added to the resulting emulsion with stirring at 400 rpm, 50 °C, pH was adjusted to 6.0 with 5% aqueous sodium hydroxide, and the resulting reaction solution was heated to reflux. After distilling off the vapor containing alcohol, the reflux was further continued for 2 hours with stirring at 400 rpm.

The resulting reaction solution was slowly cooled to room temperature with stirring at 100 rpm and solid concentration was adjusted to 60% by adding water to obtain 1010g of microcapsules containing dimethylpolysiloxane and having capsule wall made of a co-polycondensate of the silylated hydrolyzed pea protein, methyltriethoxysilane and phenyltriethoxysilane.

Reference Example 3: Production of microcapsules having capsule wall made of a co-polycondensate of silylated hydrolyzed wheat protein, methyltriethoxy silane and octyltriethoxysilane and containing squalane

Into a 1 liter beaker, 25% aqueous solution of wheat protein hydrolyzate (hydrolysed wheat protein) 100g (0.035 mol as moles calculated from amino nitrogen content) having average polymerization degree of amino acids of 7 determined from the total nitrogen content and the amino nitrogen content was charged and pH was adjusted to 9.5 by addition of 25% aqueous sodium hydroxide.

The resulting solution was warmed to 50 °C. To the warmed solution, 3-glycidoxypropyltriethoxysilane 9.5g (0.035 mol, equimolar amounts of the amino nitrogen content of the hydrolyzed wheat protein) was added dropwise over about 1 hour with stirring. After completion of the dropwise addition, stirring was continued for 14 hours at 50 °C. Thereafter, 17% aqueous hydrochloric acid was added to adjust to pH6.0 and 106g of an aqueous solution of N-[2-hydroxy-3-(3'-trihydroxysilyl)propoxy]propyl] hydrolyzed wheat protein (silylated hydrolyzed wheat protein) having the solid concentration of 24.3% was obtained.

The extent of the reaction calculated based on the amino nitrogen contents before and after the reaction was 85%. As the result, moles of N-[2-hydroxy-3-[3'-(trihydroxysilyl)propoxy]propyl] hydrolyzed wheat protein was calculated and determined to be 0.03.

Next, the aqueous solution of the silylated hydrolyzed wheat protein, thus prepared, was charged into a 2 liter glass lid circular reactor. Then, 109g of water was added to adjust the solid concentration to 120, and 17% aqueous hydrochloric acid was added to adjust to pH2.2.

The resulting solution was warmed to 50 °C and to the solution, a mixture of methyltriethoxysilane 12g and octyltriethoxysilane 3.7g was added dropwise with stirring over about 30 minutes. After the completion of the dropwise addition, stirring was continued for 4 hours at 50 °C. Then, an aqueous sodium hydroxide was added dropwise to adjust pH of the solution to 6.0, and thereto, squalane 331g was added dropwise over 2.5 hours with stirring at 600 rpm, 50 °C. Thereafter, the solution was stirred at 10,000 rpm using a homomixer at 50 °C, and finely emulsified.

Next, for the surface treatment of capsule wall, trimethylchlorosilane 1.9g was added to the resulting emulsion with stirring at 400 rpm, 50 °C, pH was adjusted to 6.0 with 5% aqueous sodium hydroxide, and the resulting reaction solution was heated to reflux. After distilling off the vapor containing alcohol, the reflux was further continued for 2 hours with stirring at 400 rpm.

The resulting reaction solution was slowly cooled to room temperature with stirring at 100 rpm and solid concentration was adjusted to 60% by adding water to obtain 613g of aqueous dispersion of microcapsules containing squalane and having capsule wall made of a co-polycondensate of the silylated hydrolyzed wheat protein, methyltriethoxysilane and octyltriethoxysilane.

Reference Example 4: Production of microcapsules having capsule wall made of a co-polycondensate of silylated hydrolyzed soy protein, methyltriethoxy silane and octyltriethoxysilane and containing water

Into a 1 liter beaker, 25% aqueous solution of soy protein hydrolyzate (hydrolysed soy protein) 100g (0.03 mol as moles calculated from amino nitrogen content) having average polymerization degree of amino acids of 5.5 determined from the total nitrogen content and the amino nitrogen content was charged and pH was adjusted to 9.5 by addition of 25% aqueous sodium hydroxide.

The resulting solution was warmed to 50 °C. To the warmed solution, 3-glycidoxypropyltriethoxysilane 7.5g (0.03 mol, equimolar amounts of the amino nitrogen content of hydrolyzed soy protein) was added dropwise over about 1 hour with stirring and stirring was continued for 14 hours at 50 °C after completion of the dropwise addition.

Thereafter, 17% aqueous hydrochloric acid was added to adjust to pH6.0 and 110g of an aqueous solution of N-[2-hydroxy-3-(3'-dihydroxymethylsilyl)propoxy]propyl ]hydrolyzed soy protein (silylated hydrolyzed soy protein) having the solid concentration of 24.5% was obtained. The extent of the reaction calculated based on the amino nitrogen contents before and after the reaction was 86%. As the result, moles of N-[2-hydroxy-3-[3'-(dihydroxymethylsilyl)propoxy] propyl]hydrolyzed soy protein was calculated and determined to be 0.03.

Next, the aqueous solution obtained above was charged into a 2 liter glass lid circular reactor, 24.7g of water was added to adjust the solid concentration to 20%, and 17% aqueous hydrochloric acid was added to adjust to pH2. 0.

The resulting solution was warmed to 50 °C and to the solution, a mixture of methyltriethoxysilane 21.4g (0.12 mol) and octyltriethoxysilane 66.2g (0.24 mol) was added dropwise with stirring over about 30 minutes. After the completion of the dropwise addition, stirring was continued for 16 hours at 50 °C.

Then, the stirring speed was increased to 600 rpm, caprylic/capric Triglyceride 214g to be the continuous phase (the outer phase the capsules disperse in) was added with stirring, stirring was continued until the phases inverted, 20% aqueous sodium hydroxide was added to adjust to pH6, and stirring was further continued at 50 °C for 3 hours at 600 rpm.

Next, a mixture of trimethylchlorosilane 3.3g (0.03 mol) and trimethylethoxysilane 7.1g (0.26 mol) was added with stirring at 600rpm, 50 °C, and then stirring was continued for 16 hours at 600rpm, 50 °C. To the resulting reaction solution, 5% aqueous sodium hydroxide was added dropwise to adjust pH of the solution to 6. The stirring speed was decreased to 400 rpm and the resulting reaction solution was gradually heated to 80 °C and kept under reflux for 2 hours to remove generated alcohol and water present in the continuous phase. Then, with stirring at 400 rpm, the temperature of the reaction solution was lowered to room temperature, and 410g of dispersion of microcapsules with about 50% water content in caprylic/capric Triglyceride was obtained (calculated content of the capsules is 50%.).

Analysis methods (test methods) will be described below which were used for evaluation of the microcapsules produced in Examples 1-12 and Reference examples 1-4 during and after their manufacturing process.

Analysis method 1: Solid concentration

About 10g of the obtained dispersion of microcapsules was weighed accurately, and water content in the dispersion of the microcapsules containing core material was measured with infrared type water content meter LIBROR EB-280MOC (trade name) manufactured by Shimadzu Corp. From the results, mass of the non-aqueous portion of the obtained microcapsule dispersion [microcapsules containing core material + free core material (core material not incorporated in capsules) + ash] is determined.

That is, in the case of oil-in-water capsules, the weight of the microcapsule dispersion is the mass of "water + microcapsule containing core material + free core material + ash", and therefore, the mass of the nonaqueous portion can be determined by the measurement of water content.

Analysis method 2: Ash content

With ICP emission spectrophotometer SPS1700HVR (trade name) manufactured by Seiko Denshi Kogyo Co., Ltd., concentration of Na in the produced microcapsule dispersion was measured, and mass of NaCl in the dispersion was calculated. It is considered that most of the ash other than silica is NaCl, and, therefore, the NaCl amount is used as ash content for estimating the core weight ratio.

Analysis method 3: Core material amount (total amount)

When the core material is an oily substance, 0.1g of obtained dispersion of microcapsules containing the core material was weighed accurately, and thereto, 5 mol/L aqueous sodium hydroxide 5mL was added. The resulting dispersion was stirred at 50 °C over 1 hour to destroy the capsule wall. After cooling to room temperature, the resultant was transferred to a 500 mL separatory funnel, while being washed with about 100mL of water, followed by shaking well after adding n-hexane 100mL and then allowed to stand still.

After the liquid phase separated into two layers, the n-hexane layer was transferred into another container. This operation was repeated three times, and the resulting n-hexane layers were combined and concentrated to make the amount to exactly 100 mL. An aliquot of the n-hexane solution was analyzed by liquid chromatography, and the core material amount contained in the microcapsules and in the continuous layer of the dispersion (the total amount of the core material) was determined from a calibration curve constructed by using standard solutions prepared separately.

Analysis method 4-1: Amount of free core material

When the core material is an oily substance, 1g of obtained dispersion of microcapsules containing core material was weighed accurately, and the dispersion was transferred to a 500mL separatory funnel, while being washed with about 100mL of water, followed by shaking well with adding n-hexane 100mL and then allowed to stand still. After the liquid phase separated into two layers, the n-hexane layer was transferred into another container. This operation was repeated three times, and the resulting n-hexane layers were combined and concentrated to make the amount to exactly 100 mL. An aliquot of the n-hexane solution was analyzed by liquid chromatography, and the core material amount present in the continuous layer of the obtained dispersion, that was the amount of the core material which was not contained into capsules (amount of free core material) was determined from a calibration curve constructed by using standard solutions prepared separately. In this specification, the amount of free core material was represented as a percentage of the amount of free core material in the total amount of the dispersion including the microcapsules.

Analysis method 4-2: Leaching rate of core material

The increase of the amount of the free core material in a period can be measured by measuring the amount of the free core material again after a certain period (e.g. after 1 day, after 1 month), and leaching rate of core material can be determined by the following numerical formula. In Examples and Comparative Examples, increase of percentage value in 30 days(%/month) was calculated and shown as the leaching rate of core material.

[Numerical formula 1]

$$\text{Leaching Rate} = \frac{\left(\begin{array}{l}\text{Measured value after}\\ \text{a certain period of time}\end{array}\right) - \left(\begin{array}{l}\text{Measured value}\\ \text{immediately after preparation}\end{array}\right)}{\text{Elapsed time}}$$

From anaylsis of the Analysis 1 to 3 and 4-1 above, it is possible to determine the core weight ratio by the following numerical formula.

[Numerical formula 2]

$$\text{Core weight ratio (\%)} = \frac{\begin{pmatrix}\text{Measured value in}\\ \text{Analysis method 3}\end{pmatrix} - \begin{pmatrix}\text{Measured value in}\\ \text{Analysis method 4-1}\end{pmatrix}}{\begin{pmatrix}\text{Measured value in}\\ \text{Analysis method 1}\end{pmatrix} - \begin{pmatrix}\text{Measured value in}\\ \text{Analysis method 2}\end{pmatrix} - \begin{pmatrix}\text{Measured value in}\\ \text{Analysis method 4-1}\end{pmatrix}} \times 100$$

Analysis method 5: Particle size distribution of capsules

Particle size distribution of capsules was measured by using SALD-2000 (trade name) manufactured by Shimadzu Corp. In this analyzer, the average particle size diameter and the standard deviation of the particle size distribution were shown. Small standard deviation means narrow distribution width of particle size.

Analysis 6: Microscopic observation

Spherical state and particle diameter of the capsules were observed by JSM-6010LA type electron microscope manufactured by JEOL Ltd. Further, in the manufacturing process of capsules, whether the capsules were formed or not was determined by optical microscopy observation (100-1000 fold).

Based on the above analysis results, the obtained microcapsules in Examples 1-12 and those in Reference Examples 1-4 were compared, and the results are shown in Table 1. In the Table, "free amount" is the amount of free core material, and "leaching rate" is the rate of leaching of core material. Since the contained material of the microcapsules of Example 12 and Reference Example 4 was water, it was not possible to analyze the free amount and the rate of leaching of the core material. The core weight ratios of Example 12 and Reference Example 4 are estimated values obtained by calculation, and they are appended mark * in the Table.

[Table 1]

| Microcapsule | Particle size distribution (Volume distribution) | | Core weight ratio (%) | Free amount (%) | Leaching rate (% /M) |
|---|---|---|---|---|---|
| | Average particle size diameter ($\mu$m) | Standard deviation | | | |
| Product of Example 1 | 1.553 | 0.227 | 90.1 | 0.000 | 0.095 |
| Product of Example 2 | 2.276 | 0.174 | 89.7 | 0.000 | 0.352 |
| Product of Example 3 | 1.557 | 0.133 | 89.9 | 0.048 | 0.034 |
| Product of Example 4 | 1.877 | 0.160 | 87.8 | 0.123 | 0.065 |
| Product of Example 5 | 2.498 | 0.176 | 87.8 | 0.029 | 0.052 |
| Product of Example 6 | 2.379 | 0.225 | 89.8 | 0.000 | 0.137 |
| Product of Example 7 | 2.563 | 0.235 | 89.8 | 0.000 | 0.371 |
| Product of Example 8 | 1.464 | 0.196 | 89.9 | 0.012 | 0.043 |
| Product of Example 9 | 2.781 | 0.201 | 88.4 | 0.571 | 0.403 |
| Product of Example 10 | 2.484 | 0.196 | 88.5 | 0.374 | 0.367 |
| Product of Example 11 | 2.988 | 0.238 | 89.0 | 0.963 | 0.387 |

(continued)

| Microcapsule | Particle size distribution (Volume distribution) | | Core weight ratio (%) | Free amount (%) | Leaching rate (% /M) |
|---|---|---|---|---|---|
| | Average particle size diameter (μm) | Standard deviation | | | |
| Product of Example 12 | 0.554 | 0.120 | 48.5* | - | - |
| Product of Reference Example 1 | 3.246 | 0.245 | 88.3 | 3.871 | 0.870 |
| Product of Reference Example 2 | 3.133 | 0.302 | 87.9 | 3.888 | 0.970 |
| Product of Reference Example 3 | 4.091 | 0.335 | 88.8 | 4.212 | 0.914 |
| Product of Reference Example 4 | 0.643 | 0.132 | 51.5* | - | - |

As shown in Table 1, microcapsules having capsule wall made of a co-polycondensate of silylated amino acid and silane compound produced in Examples 1-11 show smaller standard deviations in the particle size distribution compared to microcapsules having capsule wall made of a co-polycondensate of silylated peptide and silane compound produced in Reference Examples 1, 2 and 3.

As for the capsules containing water, a smaller standard deviation in the particle size distribution is shown in Example 12 than in Reference Example 4. That is, in Examples 1-12, microcapsules having narrow particle size distribution width were produced.

Further, the amounts of free core material and leaching rates of core material of the microcapsules containing oily substances produced in Examples 1-11 were smaller than those of the microcapsules containing oily substances produced in Reference Examples 1-3.

From this result, it is obvious that microcapsules having capsule wall made of a co-polycondensate of silylated amino acid and silane compound produced in Examples 1-11 are better in core material uptake and cause much less leaching of the core material, probably due to dense capsule wall, compared to microcapsules having capsule wall made of a co-polycondensate of silylated peptide and silane compound produced in Reference Examples 1-3.

Regarding microcapsules containing oily substances produced in Examples 1-11, pH stability and stability in the presence of a cationic substance were examined and odor was evaluated sensory. Evaluation methods and evaluation criteria are shown below. As comparative products, microcapsules having capsule wall made of a co-polycondensate of silylated peptide and silane compound produced in Reference Example 1-3 were used.

[PH stability test]

Aqueous dispersions of microcapsules having the solid concentration of 60 mass% were diluted 10-fold with water, and pH were adjusted to 3, 4 and 5 with aqueous hydrochloric acid of 1 mass% concentration. The resulting solutions were allowed to stand for 2 days at room temperature, and the solution states after 2-day standing were visually observed and evaluated on the following evaluation criteria. The results are shown with the evaluation results of stability in the presence of a cationic substance and odor in Table 2.

Evaluation criteria of PH stability

| | |
|---|---|
| Capsules sedimentate and aqueous phase is separated on upper part of the dispersion: | +++ |
| Capsules sedimentate a little and small amount of aqueous phase is separated: | ++ |
| Capsules sedimentate somewhat but aqueous phase is not separated: | + |
| No change and same as before pH adjustment: | - |

[Stability test in the presence of a cationic substance] (compatibility with cationic substance)

An aqueous dispersion of the microcapsules having a solid concentration of 60 mass% was diluted 10-fold with water. To 10 g of the 10-fold diluted dispersion, 0.5g of a mixture of stearyl trimethylammoniumchloride, water and isopropanol at mass ratio of 25: 69: 6 [Catinal STC-25W (trade name); Kao Corporation Ltd.] was added. The resulting solution was stirred well, and allowed to stand at room temperature for 2 days. The solution state after 2-day standing was visually observed and evaluated on the following evaluation criteria.

Evaluation criteria of stability in the presence of a cationic substance

| | |
|---|---|
| Capsules sedimentate and aqueous phase is separated on upper part of the dispersion: | +++ |
| Capsules sedimentate a little but aqueous phase is not separated: | ++ |
| Capsules sedimentate somewhat but aqueous phase is not separated: | + |
| No change and same as before mixing: | - |

[Evaluation of odor of microcapsule dispersion]

An aqueous dispersion of the microcapsules having a solid concentration of 60 mass% was diluted 2-fold with water, and warmed to 40 °C. The odor was evaluated on the following evaluation criteria by 10 panelists, and the average of the ten was calculated as the evaluation value of odor.

Evaluation criteria of odor

| | |
|---|---|
| Odor is not at all care about: | 3 |
| Odor is not care about: | 2 |
| Odor is somewhat bothersome (Feel somewhat): | 1 |
| Odor is very bothersome (Feel strongly): | 0 |

[Table 2]

| Microcapsule | pH stability | | | Compatibility with cationic substance | Evaluation of Odor |
|---|---|---|---|---|---|
| | 3 | 4 | 5 | | |
| Product of Example 1 | - | - | - | - | 2.5 |
| Product of Example 2 | - | - | - | < | 2.4 |
| Product of Example 3 | ++ | - | - | ++ | 2.5 |
| Product of Example 4 | + | - | - | ++ | 2.5 |
| Product of Example 5 | + | + | - | - | 2.3 |
| Product of Example 6 | + | + | - | - | 2.4 |
| Product of Example 7 | - | - | - | - | 2.3 |
| Product of Example 8 | + | - | - | + | 2.3 |
| Product of Example 9 | - | - | - | - | 2.0 |
| Product of Example 10 | - | - | - | - | 2.1 |
| Product of Example 11 | ++ | - | - | ++ | 2.4 |
| Product of Reference Example 1 | ++ | + | - | + | 1.2 |
| Product of Reference Example 2 | ++ | + | - | ++ | 0.3 |

(continued)

| Microcapsule | pH stability | | | Compatibility with cationic substance | Evaluation of Odor |
|---|---|---|---|---|---|
| | 3 | 4 | 5 | | |
| Product of Reference Example 3 | +++ | ++ | + | +++ | 0.2 |

Regarding the stability in acidic pH,

all of microcapsules of Examples had no problem in pH5 or higher,
microcapsules produced in Examples 5 and 6 became sediments a little in pH 4 and
microcapsules produced in Examples 3 and 11 were aggregated and sedimented and microcapsules produced in Examples 4, 5, 6 and 8 sedimented somewhat in pH 3.

In contrast, microcapsules of Reference examples 1-3 aggregated and were in an almost unusable state in pH4 or lower. Thus, it is evident that the microcapsules of Examples have higher stability at acidic pH.

In Examples 3, 4, 8 and 11, a silylated acidic amino acid was used, in Examples 5 and 6, a silylated basic amino acid was used, and in these Examples, the produced microcapsules were slightly poor in pH stability. Microcapsules produced by using a silylated acidic amino acid or a silylated basic amino acid seemed to be somewhat vulnerable to pH change compared with microcapsules produced by using a silylated neutral amino acid. Therefore, it is contemplated that, in a formulation where low pH is required, high stability can be achieved by using microcapsules having capsule wall made of a co-polycondensate of silylated neutral amino acid and silane compound.

The test results regarding compatibility with the cationic substance showed that, among the microcapsules produced in Examples 1-11, microcapsules produced in Examples 3, 4, 8 and 11 where an acidic amino acid, aspartic acid or glutamic acid, was utilized for the silylated amino acid, are somewhat poor in compatibility with the cationic substance, but the other products in the Examples are stable without causing turbidity or precipitation by associating with the cationic substance.

On the contrary, the microcapsules produced in Reference Examples 1-3 by using silylated peptides, caused vigorous aggregation with the cationic substance and sedimentation of the microcapsules occurred.

Regarding odor of microcapsule dispersion, evaluation values of the microcapsules produced in Examples 1-11 were values of 2 or more, while lower evaluation values were given to the microcapsules produced in Reference Examples 1-3 using silylated peptides. Thus, it was considered that the use of the microcapsules produced in Reference Examples 1-3 in cosmetics may be restricted.

Next, examples of cosmetics will be shown. In the tables showing the formulations of Examples and Comparative Examples, the amount of each component is represented by part by mass and, when amount of a component is not net solid content, solid concentration of the component is shown in parentheses after the component name.

Example 13 and Comparative Examples 1-2

The sunscreen cream of the composition shown in Table 3 was prepared to measure SPF and the feeling of use was evaluated. In Example 13, the aqueous dispersion of microcapsules having capsule wall made of a co-polycondensate of silylated serine and methyl triethoxysilane and containing 2-ethylhexyl p-methoxycinnamate as ultraviolet absorber, prepared in Example 1, was used, and

in Comparative example 1, the aqueous dispersion of microcapsules having capsule wall made of a co-polycondensate of silylated hydrolyzed casein and methyl triethoxysilane and containing 2-ethylhexyl p-methoxycinnamate, prepared in Reference Example 1, was used.

Further, in Comparative Example 2, 2-ethylhexyl p-methoxycinnamate was used as ultraviolet absorber, in place of microcapsules containing the ultraviolet absorber, and in combination with polyoxyethylene (20) oleyl ether for emulsification of the ultraviolet absorber. In the table, methyltriethoxysilane is referred to as silane compound.

[Table 3]

| | Example 13 | Comparative Example 1 | Comparative Example 2 |
|---|---|---|---|
| Aqueous dispersion of microcapsules having capsule wall made of a copolycondensate of silylated serine and silane compound and containing a UV absorber, prepared in Example 1 (60%) | 20.0 | 0.0 | 0.0 |

(continued)

| | Example 13 | Comparative Example 1 | Comparative Example 2 |
|---|---|---|---|
| Aqueous dispersion of microcapsules having capsule wall made of a copolycondensate of silylated hydrolyzed casein and silane compound and containing a UV absorber, prepared in Reference Example 1 (60%) | 0.0 | 20.0 | 0. 0 |
| 2-Ethylhexyl p-methoxycinnamate | 0.0 | 0.0 | 10.8 |
| Polyoxyethylene (20) oleyl ether | 0.0 | 0.0 | 2.5 |
| Isononyl isononanoate | 6.0 | 6.0 | 6.0 |
| Self-emulsifying type glyceryl stearate | 2 . 0 | 2 . 0 | 2 . 0 |
| Cetearyl alcohol | 2.0 | 2.0 | 2.0 |
| Sorbitan stearate | 1.0 | 1.0 | 1.0 |
| Xanthan gum | 0.2 | 0.2 | 0.2 |
| Mixture of Sodium acrylate/ sodium ac ryloyl dimethyltaurine copolymer, Isohexadecane,Polysorbate80 and Water *1 | 1.0 | 1.0 | 1.0 |
| 1, 3-Butylene glycol | 2.0 | 2 . 0 | 2 . 0 |
| Mixture of Paraoxybenzoic acid esters , Ethoxydiglycol and Phenoxyethanol *2 | 0.2 | 0.2 | 0.2 |
| Purified water | Amount making a total of 100 | Amount making a total of 100 | Amount making a total of 100 |
| In Table 3, *1 is SIMULGEL EG (trade name) manufactured by SEPPIC, and *2 is Seisept-H (trade name) manufactured by SEIWA KASEI COMPANY, LIMITED | | | |

SPF value of the sunscreen cream was measured by SPF analyzer Labsphere UV-2000S (trade name) manufactured by Labsphere, Inc. Further, each of the sunscreen cream was applied on the skin, and then smoothness and stickiness were evaluated on the following evaluation criteria and odor was evaluated on the same evaluation criteria as the criteria described in the above [Evaluation of odor of microcapsule dispersion] by 10 panelists. These results are shown in Table 4. The result of sensory evaluation is represented as the average of ten evaluation values.

Evaluation criteria of Smoothness

Very smooth: 2
Slightly smooth : 1
Grainy: 0

Evaluation criteria of stickiness

No sticky feeling: 2
Slightly sticky: 1
Very sticky: 0

[Table 4]

| | Example 13 | Comparative Example 1 | Comparative Example 2 |
|---|---|---|---|
| SPF value | 29 | 21 | 22 |
| Smoothness after application | 1.8 | 1.7 | 1.0 |
| Stickiness | 1.7 | 1.0 | 0.3 |
| Odor | 2.5 | 1.2 | 0.2 |

As shown in Table 4, the sunscreen cream of Example 13 with which microcapsules having capsule wall made of a co-polycondensate of silylated serine and silane compound and containing 2-ethylhexyl p-methoxycinnamate blended, showed higher SPF value than the sunscreen cream of Comparative example 1 blended with microcapsules having capsule wall made of a co-polycondensate of silylated hydrolyzed casein and silane compound and containing 2-ethylhexyl p-methoxycinnamate and the sunscreen cream of Comparative example 2 blended with the ultraviolet absorber without encapsulation.

Regarding the feeling of use, the sunscreen cream of Examples 13, where the ultraviolet absorber was encapsulated by capsule wall made of the a co-polycondensate of silylated amino acid and silane compound, was evaluated as excellent in smoothness, less stickiness and less odor, as compared with the sunscreen cream of Comparative Example 2, where the ultraviolet absorber was not encapsulated. In comparison with the sunscreen cream of Comparative Example 1, where the ultraviolet absorber was encapsulated by capsule wall made of a co-polycondensate of silylated peptide and silane compound, almost the same result of the evaluation was obtained in smoothness but excellent result was obtained in stickiness. This result shows that the hydrolyzed protein constituting the capsule wall is easier to cause stickiness than amino acid.

Example 14 and Comparative Examples 3-4

Milky lotions having the composition shown in Table 5 were prepared and stickiness during use, spreadability and smoothness were evaluated. In Example 14, the aqueous dispersion of microcapsule having capsule wall made of a co-polycondensate of silylated glycine, methyltriethoxysilane and phenyltriethoxy silane and containing dimethylpolysiloxane, prepared in Example 9, was used,

in Comparative example 3, the aqueous dispersion of microcapsules having capsule wall made of a co-polycondensate of silylated hydrolyzed pea protein, methyltriethoxysilane and phenyltriethoxysilane and containing dimethylpolysiloxane, prepared in Reference Example 2, was used, and

in Comparative Example 4, dimethylpolysiloxan was used as it is, in place of the microcapsules containing dimethylpolysiloxan.

[Table 5]

| | Example 14 | Comparative Example 3 | Comparative Example 4 |
|---|---|---|---|
| Aqueous dispersion of microcapsules having capsule wall made of a copolycondensate of silylated glycine and silane compound, and containing dimethylpolysiloxane, prepared in Example 9 (60%) | 15.0 | 0.0 | 0.0 |
| Aqueous dispersion of microcapsules having capsule wall made of a copolycondensate of silylated hydrolyzed pea protein and silane compound, and containin g dimethylpolysiloxane, prepared in Reference example 2 (60%) | 0.0 | 15.0 | 0.0 |
| Dimethylpolysiloxane *3 | 0.0 | 0.0 | 8.1 |
| Carboxyvinyl polymer neutralized product(0.5%) | 30.5 | 30.5 | 30.5 |
| 1. 3-Butylene glycol | 5.0 | 5.0 | 5.0 |
| Mixture of Paraoxybenzoic acid esters, Ethoxydiglycol and Phenoxyethanol *2 | 0.2 | 0.2 | 0.2 |

(continued)

| | Example 14 | Comparative Example 3 | Comparative Example 4 |
|---|---|---|---|
| Purified water | Amount making a total of 100 | Amount making a total of 100 | Amount making a total of 100 |
| In Table 5, *2 is Seisept-H (trade name) manufactured by SEIWA KASEI COMPANY, LIMITED and *3 is KF-96A-1000cs (trade name) manufactured by Shin-Etsu Chemical Co., Ltd. | | | |

The milky lotions of Example 14 and Comparative Examples 3 and 4 were evaluated by 10 panelists, picking up each of milky lotion to their hands and applying the emulsions to their faces. The spreadability of the lotions was evaluated on the following evaluation criteria and stickiness and smoothness were evaluated on the same evaluation criteria as those in Example 13. These results are shown in Table 6, represented as the average value of ten.

Evaluation criteria of spreadability

Good spreadability: 2
Moderate spreadability: 1
Poor spreadability: 0

[Table 6]

| | Example 14 | Comparative Example 3 | Comparative Example 4 |
|---|---|---|---|
| Spreadability | 1.7 | 1.6 | 1.0 |
| Stickiness | 1.8 | 1.0 | 0.2 |
| Smoothness | 1.6 | 1.5 | 0.5 |

As shown in Table 6, the milky lotion of Example 14 was better than that of Comparative Example 4 in spreadability, stickiness and smoothness upon application to the skin. Thus, the effect of encapsulating the oily substance was clearly confirmed. Further, in comparison with the milky lotion of Comparative Example 3, although, there was no significant difference in evaluation values for spreadability and smoothness, higher evaluation value of stickiness was obtained. This result shows that the silylated hydrolyzed protein constituting the capsule wall is easier to cause stickiness than silylated amino acid.

Example 15 and Comparative Examples 5-6

The lotion of the composition shown in Table 7 was prepared and affinity to skin and smoothness and stickiness after application on skin were evaluated. In Example 15, the aqueous dispersion of microcapsules having capsule wall made of a co-polycondensate of silylated aspartic acid and methyl triethoxysilane and containing squalane, prepared in Example 11, was used and
in Comparative example 5, the aqueous dispersion of microcapsules having capsule wall made of a co-polycondensate of silylated hydrolyzed wheat protein and methyltriethoxysilane and containing squalane, prepared in Reference example 3, was used.
Further, in Comparative Example 6, in place of the microcapsules containing squalane, squalane was used as it is, in combination with polyglyceryl monostearate as a surfactant for emulsification of squalane.

[Table 7]

| | Example 15 | Comparative Example 5 | Comparative Example 6 |
|---|---|---|---|
| Aqueous dispersion of microcapsules having capsule wall made of a copolycondensate of silylated aspartic acid and methyl triethoxysilane and containing squalane, prepared in Example 11 (60%) | 1.00 | 0.00 | 0.00 |
| Aqueous dispersion of microcapsules having capsule wall made of a copolycondensate of silylated hydrolyzed wheat protein and methyltriethoxysilane and containing squalane, prepared in Reference Example 3 (60%) | 0.00 | 1.00 | 0.00 |
| Squalane | 0.00 | 0.00 | 0.54 |
| Polyglyceryl monostearate | 0.00 | 0.00 | 1.00 |
| 1,3-Butylene glycol | 6.00 | 6.00 | 6.00 |
| Glycerin | 5.00 | 5.00 | 5.00 |
| Polyethyleneglycol 4000 | 3.00 | 3.00 | 3.00 |
| Ethanol | 7.00 | 7.00 | 7.00 |
| Mixture of Paraoxybenzoic acid esters, Ethoxydiglycol and Phenoxyethanol *2 | 2.00 | 2.00 | 2.00 |
| Purified water | Amount making a total of 100 | Amount making a total of 100 | Amount making a total of 100 |

Each of the lotions of Example 15 and Comparative Examples 5-6 was applied to face, and, spreadability on skin, stickiness and smoothness upon application were evaluated by 10 panelists according to the same criteria as in Example 13. In addition, affinity to skin upon application was evaluated according to the following evaluation criteria. The results are shown as the average value of the ten in Table 8.

Evaluation criteria of affinity to skin

Good affinity: 2
Moderate affinity: 1
Poor affinity: 0

[Table 8]

| | Example 15 | Comparative Example 5 | Comparative Example 6 |
|---|---|---|---|
| Affinity to skin | 1.8 | 1.0 | 0.3 |
| Smoothness after application | 1.7 | 1.3 | 0.4 |
| Stickiness | 1.5 | 1.2 | 0.2 |

As is apparent from the results shown in Table 8, the lotion of Example 15, in which the microcapsules having capsule wall made of a co-polycondensate of silylated aspartic acid and methyltriethoxysilane and containing squalane were blended, was confirmed to be better in smoothness and less stickiness, compared not only to the lotion of Comparative example 6 where squalane was not encapsulated, but also to the lotion of Comparative Example 5, in which the microcapsules having capsule wall made of a co-polycondensate of silylated hydrolyzed wheat protein and methyltriethoxysilane containing squalane were blended.

Example 16 and Comparative Examples 7-8

A lipstick having the composition shown in Table 9 was prepared and appearance, moisture content, smoothness upon application and moist feeling after application were evaluated. In Example 16, 50% caprylic/capric Triglyceride dispersion of microcapsules containing water prepared in Example 12 was used, and in Comparative Example 7, 50% caprylic/capric Triglyceride dispersion of microcapsules containing water prepared in Reference example 4 was used. Further, in Comparative Example 8, caprylic/capric Triglyceride and purified water was used so as to contain the same amount of water as the amount in Example 16. The amounts of water contained in the microcapsules of Example 12 as well as in the microcapsules of Reference example 4 were estimated by calculation to be 48.5% and 51.0%, respectively, as shown in Table 1, but the values were regarded as equivalent to 50%.

[Table 9]

| | Example 16 | Comparative Example 7 | Comparative Example 8 |
|---|---|---|---|
| Caprylic/capric Triglyceride dispersion of microcapsules containing water prepared in Example 12 (50%) | 9.26 | 0.00 | 0.00 |
| Caprylic/capric Triglyceride dispersion of microcapsules containing water prepared in Reference Example 4 (50%) | 0.00 | 9.26 | 0.00 |
| Purified water | 0.00 | 2.50 | 2.50 |
| (Hydrogenated rosin / diisostearic acid) glyceryl | 60.00 | 60.00 | 60.00 |
| Candelilla row | 4.00 | 4.00 | 4.00 |
| Carnauba wax | 3.00 | 3.00 | 3.00 |
| Hydrogenated palm oil | 3.00 | 3.00 | 3.00 |
| Ozokerite wax | 3.00 | 3.00 | 3.00 |
| Beeswax | 2.00 | 2.00 | 2.00 |
| Cholesteryl hydroxystearate | 2.00 | 2.00 | 2.00 |
| Titanium oxide | 2.00 | 2.00 | 2.00 |
| Isostearoyl hydrolyzed silk*4 | 1.00 | 1.00 | 1.00 |
| Lanolin alcohol | 1.00 | 1.00 | 1.00 |
| Diisostearyl malate | 1.00 | 1.00 | 1.00 |
| Mica | 1.00 | 1.00 | 1.00 |
| Alumina | 0.50 | 0.50 | 0.50 |
| Butyl hydroxy toluene | 0.05 | 0.05 | 0.05 |
| Tocopherol acetate | 0.05 | 0.05 | 0.05 |
| Red No. 201 | 1.50 | 1.50 | 1.50 |
| Red No. 202 | 1.50 | 1. 50 | 1.50 |
| Blue No. 1 | 0.10 | 0.10 | 0.10 |
| Yellow No. 1 | 2.00 | 2.00 | 2.00 |
| Caprylic/capric Triglyceride | 1.30 | 1.30 | 8.80 |
| In Table 9, *4 is Promois EF-118D (trade name) manufactured by SEIWA KASEI COMPANY, LIMITED. | | | |

The preparation of lipstick was carried out by mixing the components in the Table and heating at 80 °C, followed by defoaming and standing still. Water was separated at the bottom in Comparative Example 8 on standing. On the other hand, in Example 16 and Comparative Example 7, homogeneity was maintained and such a phenomenon was not observed. Then, each resultant mixture as it is in Example 16 or Comparative Example 7 or the upper phase of the mixture excluding water phase in Comparative Example 8 was poured into a lipstick mold, followed by cooling to room

temperature, and taken out from the mold to obtain a lipstick.

Water content of the obtained lipsticks of Example 16 and Comparative Examples 7-8 was measured with a Karl Fischer moisture meter. In addition, the lipsticks of Example 16 and Comparative Examples 7-8 were applied on the back of the hands of 10 panelists in an appropriate amount, and smoothness during application was evaluated according to the same evaluation criteria as in Example 13. Furthermore, moist feeling after application was evaluated according to the following evaluation criteria. These results are shown in Table 10, where smoothness and moist feeling are shown as average value of the ten panelists.

Evaluation criteria of moist feeling

| | |
|---|---|
| Good moist feeling: | 2 |
| Moderate moist feeling: | 1 |
| Poor moist feeling: | 0 |

[Table 10]

| | Example 16 | Comparative Example 7 | Comparative Example 8 |
|---|---|---|---|
| Water content in lipstick (%) | 4.88 | 4.82 | 0.05 |
| Smoothness during application | 1.8 | 0.7 | 0.8 |
| Moist feeling after application | 1.8 | 1.6 | 0.5 |

As shown in Table 10, water contents of the lipstick of Example 16 and Comparative Example 7 were 4.88% and 4.82%, respectively, while the water content of the lipstick of Comparative Example 8 was 0.05%. From these results, it was apparent that higher water content in lipsticks can be achieved by blending the microcapsules containing water.

Regarding smoothness during application, while the lipstick of Example 16 was evaluated as very smooth, evaluation values of Comparative Examples 7 and 8 were lower. In Comparative Example 7, the microcapsules having capsule wall made of a co-polycondensate of silylated peptide and silane compound containing water was used, of which particle size distribution was wider than that of the microcapsules used in Example 16, and that may have caused foreign-body sensation upon application.

As for the moist feeling after application, the lipstick of Example 16 blended with the microcapsule dispersion containing water had higher water content compared to the lipstick of Comparative Example 8, and that clearly imparted moist feeling to skin. Although the lipstick of Comparative Example 7 which used microcapsules having capsule wall made of a co-polycondensate of silylated peptide and silane compound and containing water was also shown to give good moist feeing after application, it seemed that the lipstick of Example 16 was superior to the lipstick of Comparative example 7, when considered with the feeling during application.

## Claims

1. A microcapsule containing core material in which the capsule wall is made of a silylated amino acid/silane compound copolymer which is a copolymer having a structural unit U represented by the following general formula (Ia), (Ib) or (Ic):

[Formula 1]

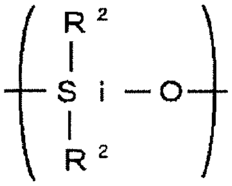

(Ia)

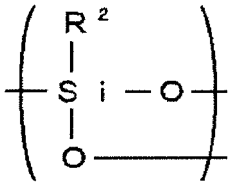

(Ib)

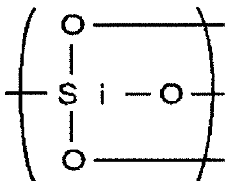

(Ic)

,

wherein, $R^2$ represents an alkyl group having 1 to 20 carbon atoms, each $R^2$ may be the same or different, and a structural unit W represented by the following general formula (Id) or (Ie):

[Formula 2]

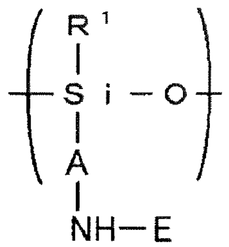

(Id)

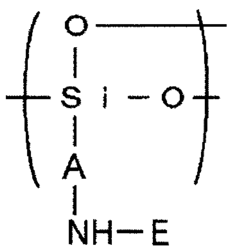

(Ie)

,

wherein, $R^1$ represents an alkyl group having 1 to 3 carbon atoms, each $R^1$ may be the same or different, A is a divalent group connecting Si and N and is at least one group selected from the group consisting of $-CH_2-$, $-CH_2CH_2-$, $-CH_2CH_2CH_2-$, $*-(CH_2)_3OCH_2CH(OH)CH_2-$ and $*-(CH_2)_3OCOCH_2CH_2-$ (* indicates the side that bonds to Si), and E is a residue obtained by removing one primary amino group from an $\alpha$ amino acid.

**2.** A microcapsule containing core material according to Claim 1, in which the structural unit U is represented by formula

(Ia) or (Ib) and the structural unit W is represented by formula (Ie).

**3.** A microcapsule containing core material according to Claim 1 or 2, in which the α amino acid is a hydrophilic α amino acid.

**4.** A microcapsule containing core material according to any one of Claims 1 to 3, in which a group represented by the following general formula (II) is bonded to the end of the silylated amino acid/silane compound copolymer;

[Formula 3]

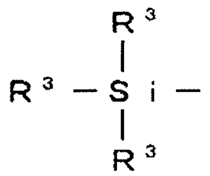

(II)

wherein, $R^3$ represents an alkyl group having 1 to 4 carbon atoms or a phenyl group, each $R^3$ may be the same or different.

**5.** A microcapsule containing core material according to any one of Claims 1 to 4, in which A in the general formula (Id) and (Ie) represents $*-(CH_2)_3OCH_2CH(OH)CH_2-$(* indicates the side that bonds to Si).

**6.** A microcapsule containing core material

which has capsule wall made of a silylated amino acid/silane compound copolymer obtained by co-polycondensing
a silylated amino acid in which a silyl group represented by the following general formula (III):

[Formula 4]

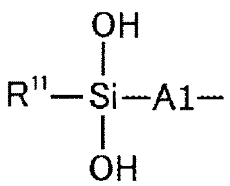

(III)

wherein, $R^{11}$ represents a hydroxyl group or an alkyl group having 1 to 3 carbon atoms, and A1 represents a connecting group selected from a group consisting of $-CH_2-$, $-CH_2CH_2-$, $-CH_2CH_2CH_2-$, $*-(CH_2)_3OCH_2CH(OH)CH_2-$ and $*-(CH_2)_3OCOCH_2CH_2-$(* indicates a side that bonds to Si), is bonded to an amino group of an alpha-amino acid
and
a silane compound represented by the following general formula (IV):

$$R^{21}{}_mSi(OH)_nY_{(4-n-m)} \quad (IV),$$

wherein, $R^{21}$ represents an alkyl group having 1 to 20 carbon atoms or a phenyl group, m is an integer from 0 to 3, all the m of $R^{21}$ may be the same or different, n is an integer from 0 to 4, $m+n \leqq 4$, (4-n-m) of Y represent a hydrogen or an alkoxy group having 1 to 6 carbon atoms,
in a dispersion in which a phase of an oily substance to become core material is dispersed in a continuous phase constituted of an aqueous substance, or
in a dispersion in which a phase of an aqueous substance to become core material is dispersed in a continuous phase constituted of an oily substance; and which contains the above-core material.

**7.** A microcapsule containing core material according to Claim 6, in which the continuous phase is an aqueous substance and the dispersed phase is an oily substance.

**8.** A microcapsule containing core material according to Claim 6 or 7, in which A1 in the general formula (III) represents *-$(CH_2)_3OCH_2CH(OH)CH_2$- (* indicates a side that bonds to Si).

**9.** A cosmetic **characterized in that** microcapsules containing core material according to any one of Claims 1 to 8 are contained.

**10.** A cosmetic according to Claim 9 **characterized in that** 0.01 mass% to 35 mass% of the microcapsules containing core material are contained.

## Patentansprüche

**1.** Eine Mikrokapsel, die ein Kernmaterial enthält, wobei
die Kapselwand aus einem silylierten Aminosäure/Silanverbindungs-Copolymer besteht, bei dem es sich um ein Copolymer handelt, das eine Struktureinheit U, dargestellt durch die folgende allgemeine Formel (Ia), (Ib) oder (Ic) aufweist:

[Formel 1]

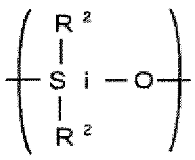
(Ia)

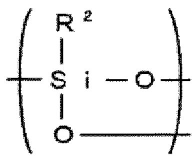
(Ib)

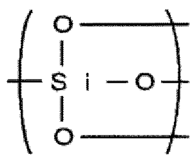
(Ic)

wobei $R^2$ eine Alkylgruppe mit 1 bis 20 Kohlenstoffatomen darstellt, jedes $R^2$ gleich oder verschieden sein kann, und
eine Struktureinheit W, dargestellt durch die folgende allgemeine Formel (Id) oder (Ie) ist:

[Formel 2]

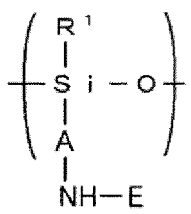
(Id)

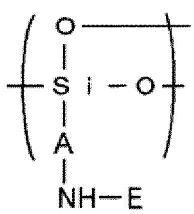
(Ie)

wobei $R^1$ eine Alkylgruppe mit 1 bis 3 Kohlenstoffatomen darstellt, jedes $R^1$ gleich oder verschieden sein kann, A eine zweiwertige Gruppe ist, die Si und N verbindet, und mindestens eine Gruppe, ausgewählt aus der Gruppe bestehend aus $-CH_2-$, $-CH_2CH_2-$, $-CH_2CH_2CH_2-$, $*-(CH_2)_3OCH_2CH(OH)CH_2-$ und $*-(CH_2)_3OCOCH_2CH_2-$, ist (* gibt die Seite an, die an Si bindet), und E ein Rest ist, der durch Entfernen einer primären Aminogruppe von einer $\alpha$-Aminosäure erhalten wird.

**2.** Eine Mikrokapsel, die ein Kernmaterial enthält, nach Anspruch 1, wobei die Struktureinheit U durch Formel (Ia) oder (Ib) dargestellt ist und die Struktureinheit W durch Formel (Ie) dargestellt ist.

**3.** Eine Mikrokapsel, die ein Kernmaterial enthält, nach Anspruch 1 oder 2, wobei die $\alpha$-Aminosäure eine hydrophile $\alpha$-Aminosäure ist.

**4.** Eine Mikrokapsel, die ein Kernmaterial enthält, nach einem der Ansprüche 1 bis 3, wobei eine Gruppe, dargestellt durch die folgende allgemeine Formel (II), an das Ende des silylierten Aminosäure/Silanverbindungs-Copolymers gebunden ist,

[Formel 3]

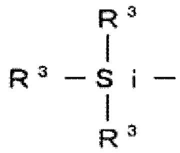

(II)

wobei $R^3$ eine Alkylgruppe mit 1 bis 4 Kohlenstoffatomen oder eine Phenylgruppe darstellt, jedes $R^3$ gleich oder verschieden sein kann.

**5.** Eine Mikrokapsel, die ein Kernmaterial enthält, nach einem der Ansprüche 1 bis 4, wobei A in der allgemeinen Formel (Id) und (Ie) $*-(CH_2)_3OCH_2CH(OH)CH_2-$ darstellt (* gibt die Seite an, die an Si bindet).

**6.** Eine Mikrokapsel, die ein Kernmaterial enthält,

welche eine Kapselwand aufweist, die aus einem silylierten Aminosäure/Silanverbindungs-Copolymer gemacht ist, das erhalten wird durch Copolykondensation
einer silylierten Aminosäure, wobei eine Silylgruppe, dargestellt durch die folgende allgemeine Formel (III):

[Formel 4]

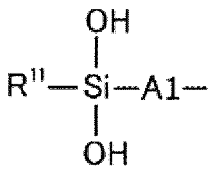

(III)

wobei $R^{11}$ eine Hydroxylgruppe oder eine Alkylgruppe mit 1 bis 3 Kohlenstoffatomen darstellt und A1 eine Verbindungsgruppe, ausgewählt aus einer Gruppe bestehend aus $-CH_2-$, $-CH_2CH_2-$,$-CH_2CH_2CH_2-$, $*-(CH_2)_3OCH_2CH(OH)CH_2-$ und $*-(CH_2)_3OCOCH_2CH_2-$ (* gibt eine Seite an, die an Si bindet), darstellt, an eine Aminogruppe einer alpha-Aminosäure gebunden ist, und
einer Silanverbindung, dargestellt durch die folgende allgemeine Formel (IV):

$$R^{21}{}_mSi(OH)_nY_{(4-n-m)} \qquad (IV),$$

wobei $R^{21}$ eine Alkylgruppe mit 1 bis 20 Kohlenstoffatomen oder eine Phenylgruppe darstellt, m eine ganze Zahl von 0 bis 3 ist, alle m des $R^{21}$ gleich oder verschieden sein können, n eine ganze Zahl von 0 bis 4 ist, $m+n \leq 4$, (4-n-m) des Y einen Wasserstoff oder eine Alkoxygruppe mit 1 bis 6 Kohlenstoffatomen darstellen,
in einer Dispersion, in der eine Phase einer öligen Substanz, die ein Kernmaterial wird, in einer kontinuierlichen Phase, die aus einer wässrigen Substanz zusammengesetzt ist, dispergiert ist, oder

in einer Dispersion, in der eine Phase einer wässrigen Substanz, die ein Kernmaterial wird, in einer kontinuierlichen Phase, die aus einer öligen Substanz zusammengesetzt ist, dispergiert ist; und die das vorstehende Kernmaterial enthält.

**7.** Eine Mikrokapsel, die ein Kernmaterial enthält, nach Anspruch 6, wobei die kontinuierliche Phase eine wässrige Substanz ist und die dispergierte Phase eine ölige Substanz ist.

**8.** Eine Mikrokapsel, die ein Kernmaterial enthält, nach Anspruch 6 oder 7, wobei A1 in der allgemeinen Formel (III) $*-(CH_2)_3OCH_2CH(OH)CH_2-$ darstellt (* gibt eine Seite an, die an Si bindet).

**9.** Ein Kosmetikum, **dadurch gekennzeichnet, dass** Mikrokapseln, die ein Kernmaterial enthalten, nach einem der Ansprüche 1 bis 8 enthalten sind.

**10.** Ein Kosmetikum nach Anspruch 9, **dadurch gekennzeichnet, dass** 0,01 Massen-% bis 35 Massen-% der Mikrokapseln, die ein Kernmaterial enthalten, enthalten sind.

## Revendications

**1.** Microcapsule contenant un matériau de coeur, dans laquelle
la paroi de capsule est faite d'un copolymère d'acide aminé silylé / composé silane qui est un copolymère ayant un motif structurel U représenté par l'une des formules générales (Ia), (Ib) ou (Ie) suivantes :

[formule 1]

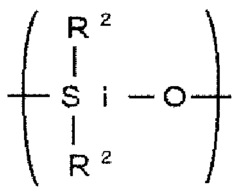

(Ia)

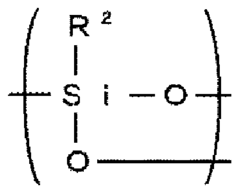

(Ib)

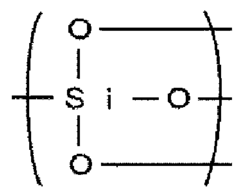

(Ic)

dans laquelle $R^2$ représente un groupe alkyle ayant 1 à 20 atomes de carbone, et les $R^2$ peuvent être identiques ou différents, et
un motif structurel W représenté par l'une des formules générales (Id) ou (Ie) suivantes :

[formule 2]

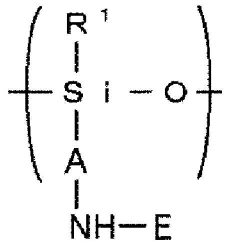

(Id)

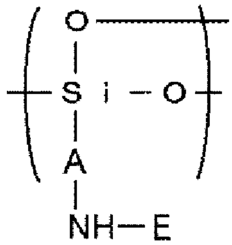

(Ie)

dans laquelle $R^1$ représente un groupe alkyle ayant 1 à 3 atomes de carbone, chacun des $R^1$ peuvent être identiques ou différents, A est un groupe divalent connectant Si et N et est au moins un groupe choisi dans l'ensemble constitué par $-CH_2-$, $-CH_2CH_2-$, $-CH_2CH_2CH_2-$, $*-(CH_2)_3OCH_2CH(OH)CH_2-$ et $*-(CH_2)_3OCOCH_2CH_2-$ (* indique le côté qui se lie à Si) et E est un résidu obtenu par élimination d'un groupe amino primaire d'un acide $\alpha$-aminé.

2. Microcapsule contenant un matériau de coeur selon la revendication 1, dans laquelle le motif structurel U est représenté par la formule (Ia) ou (Ib) et le motif structurel W est représenté par la formule (Ie).

3. Microcapsule contenant un matériau de coeur selon la revendication 1 ou 2, dans laquelle l'acide $\alpha$-aminé est un acide $\alpha$-aminé hydrophile.

4. Microcapsule contenant un matériau de coeur selon l'une quelconque des revendications 1 à 3, dans laquelle un groupe représenté par la formule générale (II) qui suit est lié à l'extrémité du copolymère d'acide aminé silylé / composé silane ;

[formule 3]

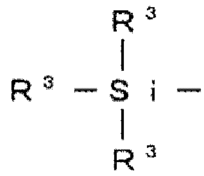

(II)

dans laquelle $R^3$ représente un groupe alkyle ayant 1 à 4 atomes de carbone ou un groupe phényle, et les $R^3$ peuvent être identiques ou différents.

5. Microcapsule contenant un matériau de coeur selon l'une quelconque des revendications 1 à 4, dans laquelle A dans les formules générales (Id) et (Ie) représente $*-(CH_2)_3OCH_2CH(OH)CH_2-$ (* indique le côté qui se lie à Si).

6. Microcapsule contenant un matériau de coeur,

qui a une paroi de capsule faite d'un copolymère d'acide aminé silylé / composé silane obtenu par copolycondensation de

un acide aminé silylé dans lequel un groupe silyle représenté par la formule générale (III) qui suit :

[formule 4]

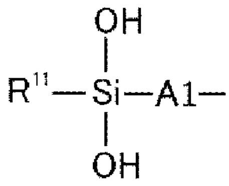

(III)

dans laquelle $R^{11}$ représente un groupe hydroxyle ou un groupe alkyle ayant 1 à 3 atomes de carbone, et A1 représente un groupe de connexion choisi parmi l'ensemble constitué par $-CH_2-$, $-CH_2CH_2-$, $-CH_2CH_2CH_2-$, $*-(CH_2)_3OCH_2CH(OH)CH_2-$ et $*-(CH_2)_3OCOCH_2CH_2-$ (* indique le côté qui se lie à Si),
est lié à un groupe amino d'un acide alpha-aminé, et
d'un composé silane représenté par la formule générale (IV) suivante :

$$R^{21}{}_mSi(OH)_nY_{(4-n-m)} \qquad (IV)$$

dans laquelle $R^{21}$ représente un groupe alkyle ayant 1 à 20 atomes de carbone ou un groupe phényle, m est un entier de 0 à 3, tous les m $R^{21}$ peuvent être identiques ou différents, n est un entier de 0 à 4, $m + n \leq 4$, les (4-n-m) Y représentent un hydrogène ou un groupe alcoxy ayant 1 à 6 atomes de carbone,
dans une dispersion dans laquelle une phase d'une substance huileuse devant devenir un matériau de coeur est dispersée dans une phase continue constituée d'une substance aqueuse, ou
dans une dispersion dans laquelle une phase d'une substance aqueuse devant devenir un matériau de coeur est dispersée dans une phase continue constituée d'une substance huileuse ; et qui contient le matériau de coeur ci-dessus.

**7.** Microcapsule contenant un matériau de coeur selon la revendication 6, dans laquelle la phase continue est une substance aqueuse et la phase dispersée est une substance huileuse.

**8.** Microcapsule contenant un matériau de coeur selon la revendication 6 ou 7, dans laquelle A1 dans la formule générale (III) représente $*-(CH_2)_3OCH_2CH(OH)CH_2-$ (* indique le côté qui se lie à Si).

**9.** Cosmétique **caractérisé en ce qu'**il contient des microcapsules contenant un matériau de coeur selon l'une quelconque des revendications 1 à 8.

**10.** Cosmétique selon la revendication 9, **caractérisé en ce qu'**il contient 0,01 % en masse à 35 % en masse des microcapsules contenant un matériau de coeur.

[Fig.1]

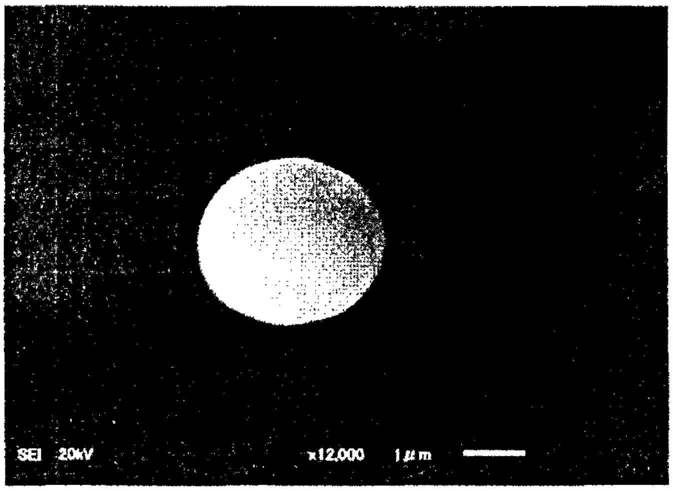


[Fig.2]

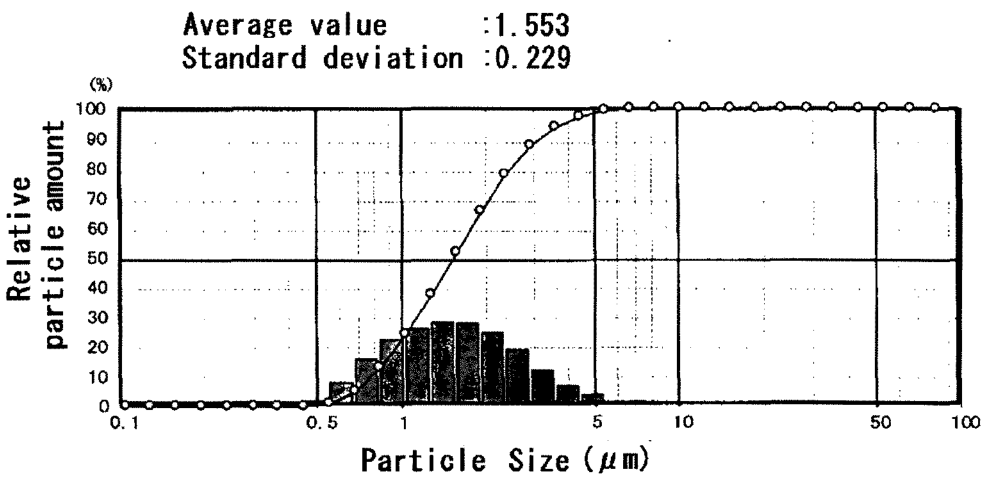


[Fig.3]

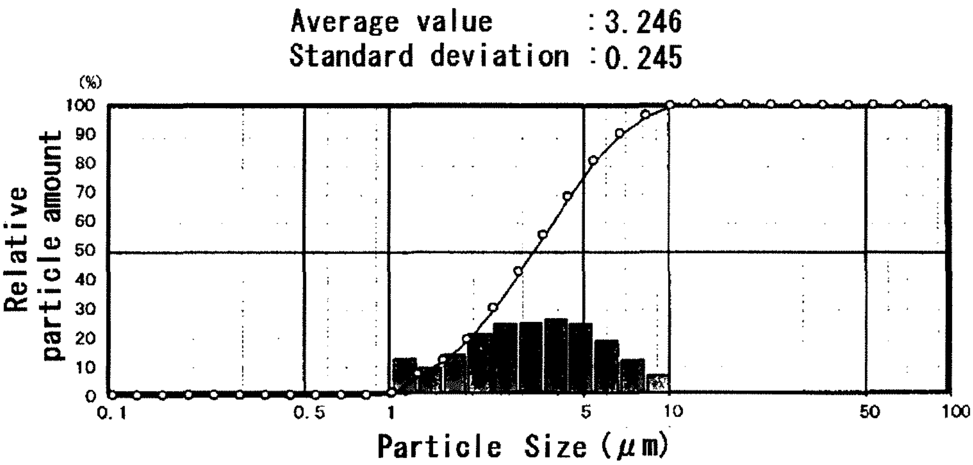
Average value : 3.246
Standard deviation : 0.245
(%)
100
90
80
70
60
50
40
30
20
10
0
Relative particle amount
0.1
0.5
1
5
10
50
100
Particle Size (μm)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002037713 A
- JP 10277512 A
- JP 11221459 A
- JP 2000225332 A
- JP 2001049233 A
- JP 8059424 A
- JP 8067608 A